# EUROPEAN PATENT APPLICATION

(11) **EP 2 957 555 A1**
(43) Date of publication of application: **23.12.2015**
(21) Application number: 14751222.2
(22) Date of filing: 11.02.2014
(51) Int. Cl.: C07C 229/24, C07C 229/28, C07C 229/36, C07C 233/30, C07C 233/34, C07C 233/46, C07D 207/27, C07D 307/33, C07K 1/107

(54) **METHOD FOR MODIFYING DERIVATIVES OF AMINO ACIDS AND PEPTIDES, COMPOUNDS PRODUCED, AND USE**

(30) Priority: 12.02.2013 ES 201330177
(71) Applicant: Consejo Superior De Investigaciones Científicas (CSIC), 28006 Madrid (ES)
(72) Inventor: ROMERO ESTUDILLO, Iván Omar, Madrid 28006 (ES); BOTO CASTRO, Alicia, 28006 Madrid (ES)
(74) Representative: Pons Ariño, Angel
(86) International application number: PCT/ES2014/070096
(87) International publication number: WO 2014/125145

(57) **Abstract**

The invention relates to a novel method for modifying hydroxylated cyclic amino acids, especially 4-hydroxyproline, which is a commercial, relatively cheap, natural amino acid, in order to provide other amino acids of high optical purity. The initial step of the process is a free-radical excision, and the excision product converts itself into non-proteinogenic amino acids by means of reactions such as reduction, reductive amination, and Horner-Wadsworth-Emmons reactions. The method is characterised in that it is applicable to the selective modification of peptides. The invention also relates to the products of said method, such as amino acids that are N-alkylated and/or have side chains with groups of amino, hydroxyl, alkenyl etc., and to the peptidic derivatives thereof of interest as pharmaceuticals, catalysts, molecular imaging probes etc.

## Description

### FIELD OF THE ART

The present invention belongs to the chemistry sector, and refers to the development of new "customizable" amino acids, and their conversion into unnatural amino acids of high optical purity, including high-profit *N*-alkyl amino acids.

Moreover, the "customizable" amino acids can be incorporated into peptides and selectively modified, even if the peptide presents other residues of the same customizable unit. This would allow the direct conversion of a commercially available peptide into many others, which could be used as chemical reagents.

The present invention also falls within the pharmaceutical sector, since some of the resulting peptides, or derivatives thereof, could serve as drugs.

### PURPOSE OF THE INVENTION

The purpose of the invention is a novel process for the modification of hydroxylated cyclic amino acids, particularly 4-hydroxyproline, which is a natural, commercial and relatively inexpensive amino acid, to give other amino acids of high optical purity, and the application of this procedure to site-selective peptide modification. The invention also refers to the products resulting from such modification, for instance *N*-alkyl amino acids or residues presenting lateral chains with amino, hydroxyl, or alkenyl groups, and their peptide derivatives, of use as drugs, probes for molecular imaging, etc

### STATE OF THE ART

The transformation of low-cost amino acids into high-profit, non-natural ones allows the preparation of new chemical reagents, drugs and medical probes, among other products.

Glycine is the most commonly used amino acid, mainly by formation of enolates and alkylation, as shown by the conversion of glycine derivative (1) (Scheme 1) into the 2-alkyl glycine (2) under phase-transfer conditions, as described by Hashimoto, T.; Maruoka, K. Chem. Rev. 2007, 107, 5656-5682, and Ooi, T.; Maruoka, K. Angew. Chem. Int. Ed. 2007, 46, 4222-4266. Less used are serine and threonine, which generally undergo dehydratation to afford dehydroamino acids, or scission-addition of new lateral chain processes, as shown by the transformation (3)→(4). Thus, Boto and Hernández have reported the scission of simple serine derivatives such as compound (3) with (diacetoxyiodo)benzene and iodine under irradiation with visible light, generating an acyliminium ion such as (3a), which was trapped by oxygen, nitrogen, sulfur or carbon nucleophiles [Boto, A.; Gallardo, J. A.; Hernández, D.; Hernández, R. J. Org. Chem. 2007, 72, 7260-7269 and also Boto, A.; Gallardo, J. A.; Hernández, R.; Ledo, F.; Muñoz, A.; Murguía, J. R.; Menacho-Márquez, M.; Orjales, A.; Saavedra, C. J. Bioorg. Med. Chem. Lett. 2006, 16, 6073-6077].

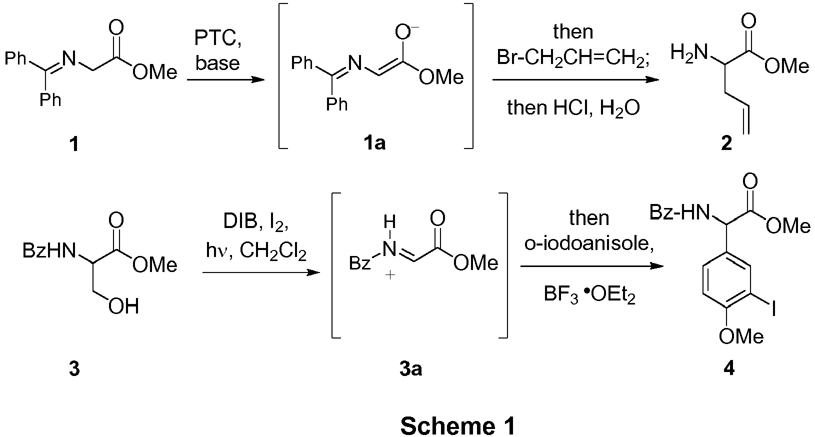

These non-natural amino acids are useful constituents of modified peptides of pharmacological interest, either by allowing the modulation of the peptide bioactivity, or by providing increased resistance to hydrolysis *in vivo* or improved bioavailability [(a) Acc. Chem. Res. 2008, 41, N°10, Special Issue on *Peptidomimetics.* (b) Sieburth, S. M.; Chen, C. A. Eur. J. Org. Chem. 2006, 311-322. (c) Chem. Rev. 2006, 106, N°7, Special Issue on *Process Chemistry*]. In addition, unnatural amino acids can serve as fluorescent or radioactive units in peptide probes for molecular imaging. All these applications have attracted attention from the pharmaceutical companies, and elicited an active research in the area.

Besides, many synthetic peptides have been developed as organocatalysts for different chemical processes, such as acylations, regio- and enantioselective phosphorylations, asymmetric epoxidation, kinetic resolutions, etc [Colby-Davie, E. A.; Mennen, S. M.; Xu, Y.; Miller, S. J. Chem. Rev. 2007, 107, 5759-5812]. The introduction of unnatural amino acids for substrate recognition or as reactive centers allowes considerable improvements in catalyst performance.

In order to develop bioactive or catalytic peptides containing unnatural amino acids, libraries of these compounds are prepared and then their structure-bioactivity relationships are studied. In the traditional process to generate libraries, each compound is synthesized *de novo,* by introduction of a modified unit with respect to the reference peptide (Scheme 2). The example shows the preparation of five analogues of a bioactive peptide aa₁-aa₂-aa₃-aa₄-aa₅ (5), where the 4-position is considered vital for modulation of the bioactivity and therefore is modified. In order to prepare the five analogues aa₁-aa₂-aa₃-XX₄-aa₅ (6-10), at least 40 synthetic steps would be needed, since the introduction of a new aaₙ unit requires two steps, namely the deprotection of the terminal residue [a] and a coupling step [b].

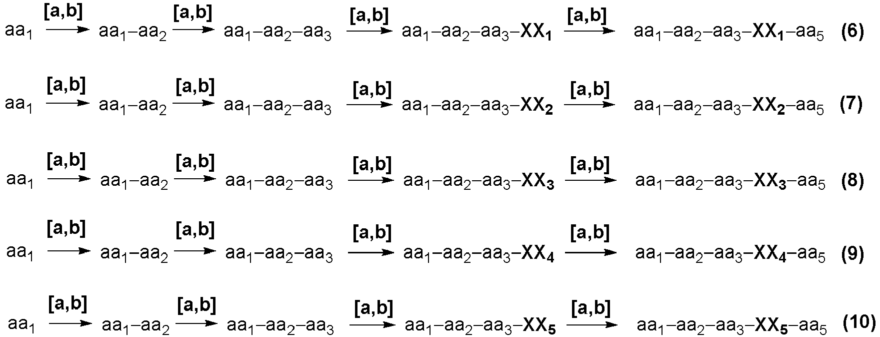

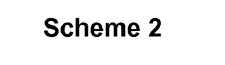

This serial procedure is costly in time and materials. An alternative strategy (Scheme 3) starts from a single peptide (or a few ones) which can be selectively manipulated in certain positions, while the others remain unaffected.

Thus, for the preparation of the five analogues (6-10), a starting peptide aa₁-aa₂-aa₃-YY₄-aa₅ (11) would be prepared and then selectively modified at the 4-position [step 3]. Ideally, [c] is a one-step reaction and thus the overall process would only need thirteen steps. Obviously, the larger the peptide to be modified, the greater the difference between the number of steps of the conventional and the site-selective modification processes.

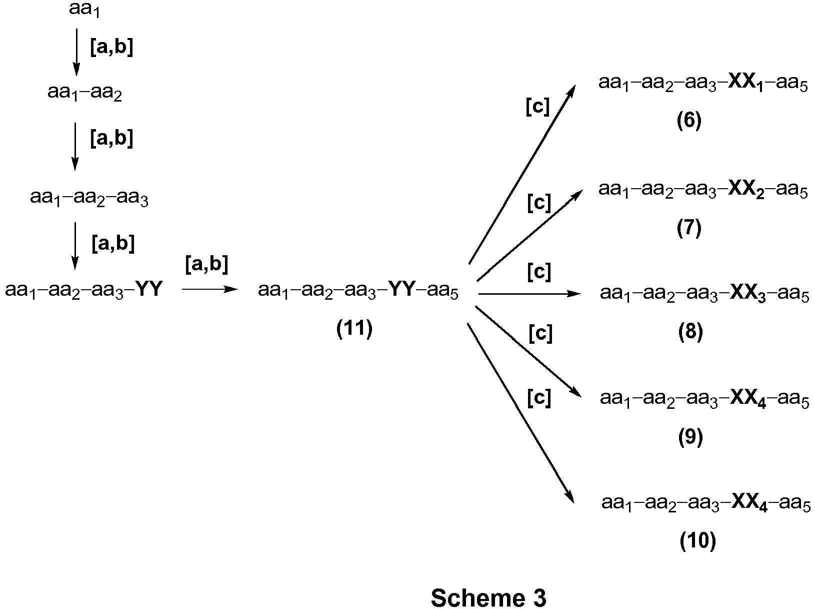

However, the selective modification of peptides is very difficult, due to the similar reactivity of the amino acid units, as commented in Qi, D .; Tann, C. M .; Distefano, M.D. Chem Rev. 2001, 101, 3081-3112 and Antos, J.M.; Francis, M. B. Curr. Opin. Chem. Biol. 2006, 10, 253-262. In fact, although many methods have been reported for the modification of simple amino acid residues, only few methods describe the site-selective modification of residues in peptides.

Most of the work reported in this area describes the modification of glycine residues. Thus, glycine lateral chain (H) has been replaced by alkyl, allyl, and other chains. For example, Seebach and Skrydstrup have generated glycine enolates at low temperatures, which were then trapped by different electrophiles. [Seebach, D.; Bech, A. K.; Studer, A. Modern Synthetic Methods, vol. 7 Eds. Ernst, B.; Leumann, C.; VCH, Weinheim, 1995, as well as Ricci, M.; Madariaga, L .; Skrydstrup, T. Angew. Chem. Int. Ed. 2000, 39, 242-245].

The procedure reported by Skrydstrup takes place under milder conditions than the method described by Seebach, but requires several reaction steps; nevertheless, good overall yields are obtained. In this method, an α--thioglycine is generated, and then converted into an enolate which is trapped by electrophiles. Steglich and Beck have reported a similar approach, but the α-thioglycine served as precursor of a glycyl cation which was trapped by nucleophiles [Dialer, H.; Steglich, W.; Beck, W. Tetrahedron 2001, 57, 4855-4861].

Kazmaier has developed an efficient site-selective modification where step [c] involves one reaction (Scheme 4) as described in Deska, J.; Kazmaier, U. Chem. Eur. J. 2007, 13, 6204-6211 and in Datta, S.; Kazmaier, U. Org. Biomol. Chem. 2011, 9, 872-880], namely the allylation or alkylation of glycine zinc enolates catalyzed by palladium complexes.

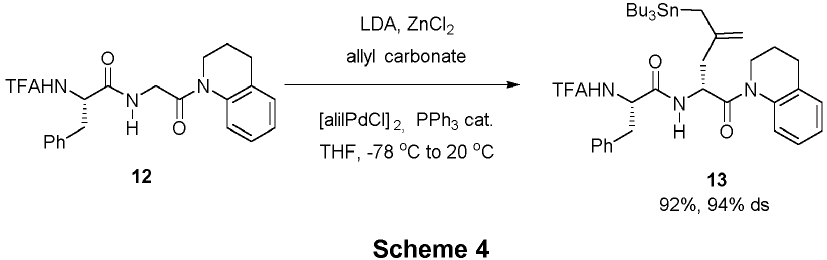

Although effective, this method presents a disadvantage: when the peptide posseses several glycine residues, the selective modification of just one residue is challenging. In fact, examples of the site-selective modification of peptides containing two or more glycine residues have not been reported.

In order to solve the problem of the site-selective modification of peptides with several similar customizable units, glycine can be replaced by serine or threonine residues. Thus, the lateral chains of these new units can be protected orthogonally, with groups that can be introduced or removed independently [Boto, A.; Hernandez, R.; Fernandez, C. Patent Appl. P201131095, Chemical process for the selective modification of peptides]. As shown in conversion (14) → (16) (Scheme 5), while serine units with free OH groups are transformed, protected residues remain unaffected. After the first transformation, another serine residue could be deprotected, and thus a second selective modification could be carried out.

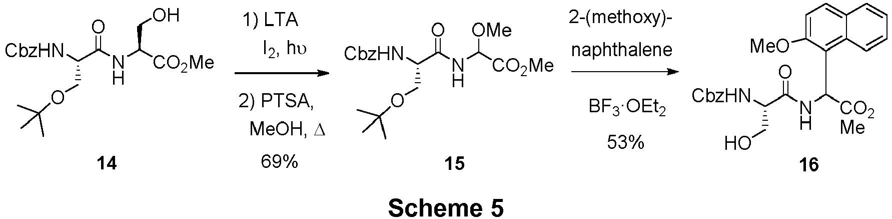

An advantage of this strategy is the commercial availability of relatively inexpensive serine derivatives. The protected derivatives may be incorporated into the starting peptide as previously commented in Scheme 3.

This methodology can be applied to peptides of different sizes, containing as many serine residues as desired, as long as these residues are orthogonally protected. The residues may occupy any position in the peptide, from the terminal to the internal positions.

There are few reports in the literature describing the selective transformation of serine or threonine residues in peptides, where the lateral chains of these residues are cleaved and replaced by other chains. Using a variation of the previous method, Boto et al [Patent Appl. P201131095, Chemical Process for the site-selective modification of peptides] carried out an oxidative radical scission of a threonine residue, followed by the addition of triethyl phosphite, as shown in the conversion 17→19 (Scheme 6) and as described in Saavedra, C. J.; Boto, A.; Hernández, R. Org. Lett., 2012, 14, 3788-3791. The resulting amino phosphonate was used in a Horner-Wadsworth-Emmons reaction to give dehydroamino acids.

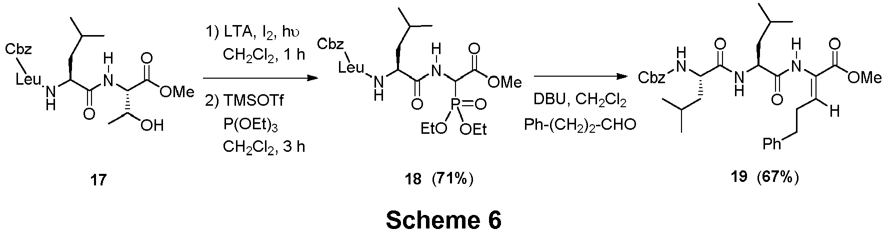

While in the method reported by Boto et al. the scission of the lateral chain generated a glycyl cation which was trapped by nucleophiles, Skrydstrup et al. carried out the scission and then generated an enolate which was trapped by electrophiles, as shown in the conversion (20)→(24) (Scheme 7) and as described in Blakskjær, P.; Gavrila, A.; Andersen, L.; Skrydstrup, T. Tetrahedron Lett. 2004, 45, 9091-9094 as well as Ebran, J. P.; Jensen, C. M.; Johannesen, S. A.; Karaffa, J.; Lindsay, K. B.; Taaning, R.; Skrydstrup, T. Org. Biomol. Chem. 2006, 4, 3553-3564.

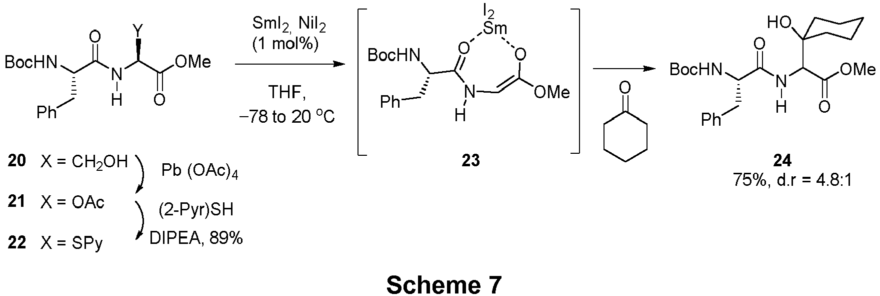

For the first step, Skrydstrup used an oxidative radical scission previously reported by Steglich [Schuemann, S.; Zeitler, K.; Jäger, M.; Polborn, K.; Steglich, W. Tetrahedron 2000, 56, 4187-4195], converting the serine unit into an α-acetoxyglycine residue, thus forming the dipeptide (21). The replacement of the acetoxy group by other oxygen, nitrogen or sulfur functions can be carried out readily, but the introduction of carbon chains under mild conditions, which do not affect other amino acid units, is quite challenging. Therefore, Skrydstrup developed a multistep process, where the acetoxy group was replaced by a thiopyridyl moiety, affording compound (22). In the presence of SmI₂, an anion (23) was generated and then trapped by electrophiles, for instance carbonyl derivatives. In this way, the serine residue can be transformed into amino acids with unnatural chains, as shown by compound (24).

Other "customizable" units used for the selective modification of peptides are glutamic acid residues, as shown by the conversion of peptide (25) into products (26)-(29) (Scheme 8), and as described in Saavedra, C. J.; Boto, A.; Hernández, R. Org. Lett., 2012, 14, 3542-3545.

This one-step decarboxylation-alkylation process allows the conversion of peptides with glutamic acid residues into α,γ-hybrid peptides, containing unnatural γ-amino acids. The process takes place under mild conditions and in good overall yields. Starting from a single peptide, a library of α,γ-hybrid peptides can be generated. Since the carboxyl groups of the glutamic acid units can be protected with orthogonal groups, the starting peptide may contain several glutamic units, but only the unprotected units will be transformed. The protecting groups can be sequentially removed, so that subsequent modifications can be carried out.

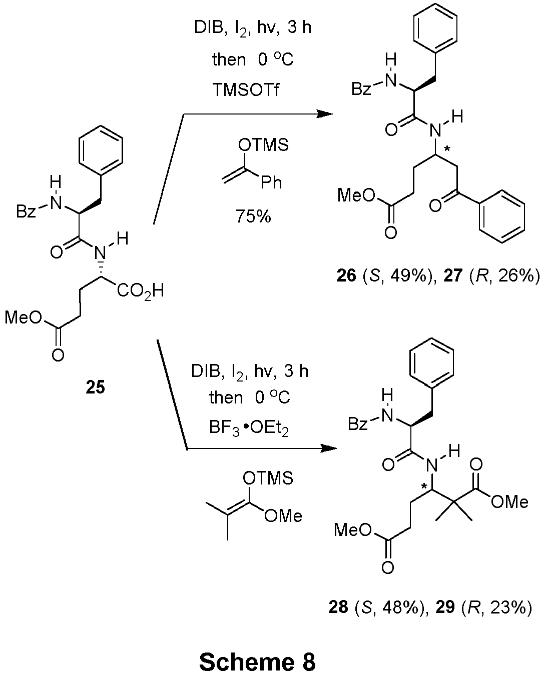

In this example, and in the previous ones, the selective modification originates epimer mixtures, but the isomers can be separated. In order to prepare optically pure products, chiral catalysts or reagents would be required, but they should be optimized for each desired product. In the present patent application an alternative strategy is presented, based on the use of "customizable" units which can undergo scission to give new amino acids with high optical purity. The latter can serve as precursors of other non-proteinogenic amino acids.

### BRIEF DESCRIPTION

The selective modification of peptides represents a difficult challenge, particularly when the peptide contains very similar customizable units. Previous methodologies have been developed for the site-selective modification of serine, threonine or glutamic acid units, even in the presence of similar residues. However, although these methods are quite efficient, the stereoselectivity of the process is not usually high. Therefore, new methods are sought that allow the generation of amino acids with high optical purity, by modification of new types of customizable units.

This Patent Application describes the use of hydroxylated cyclic amino acids, particularly 4*R*-hydroxy-L-proline, as customizable units in sequential scission-addition processes, to yield amino acids with high optical purity and a variety of unnatural lateral chains, which may contain hydroxy, amino, alkenyl or aryl groups. Moreover, the method allows extension of peptide chains, and the preparation of high-profit *N*-alkyl amino acid derivatives.

### DETAILED DESCRIPTION OF THE INVENTION

The present invention is based on the observation that some cyclic hydroxyamino acids can be modified using the procedure protected herein, forming new optically pure unnatural amino acids, and that this procedure allows the site-selective modification of peptides.

In the present invention, the term "tunable" or "customizable units" refers to an amino acid unit which can be selectively modified in the presence of other residues, which remain unaffected. In particular, in the description of the invention, the term "tunable" or "customizable units" refers to hydroxylated cyclic amino acids which, under the conditions of the protected process, undergo scission to give non-proteinogenic, optically pure acyclic amino acids.

The competitive advantages of the procedure and the use of the present invention are listed below:
1) Generation of amino acid derivatives with high optical purity.
2) Site-selective transformation of "customizable" residues in peptides, even in the presence of very similar units.
3) Mild reaction conditions, which use reagents compatible with most functional groups, particularly during the oxidative radical scission step.
4) Ready transformation of the scission products into unnatural amino acid derivatives with a variety of groups such as hydroxyl, amino, alkenyl, aryl or heteroaryl groups, and even peptidic chains.
5) Preparation of amino acids with NH or N-alkyl groups, according to the reaction conditions. The *N*-alkyl derivatives, such as the N-methyl amino acids, are high-profit products. Besides, there are not methodologies to selectively methylate a previously unactivated position in the peptide.
6) Straightforward conversion of a starting peptide into a library of derivatives, which could serve as precursors of other peptides, and therefore serve as chemical reagents.

The present invention proposes a new process for the transformation of amino acid and peptide derivatives, where hydroxylated cyclic amino acids are used as "customizable" units, in particular the commercial amino acid 4-hydroxyproline (Hyp). As shown in conversions (30)→(32-35) (Scheme 9), an oxidative radical scission would take place; it must be said that the fragmentation of a secondary alcohol such as Hyp is more favoured than the scission of a primary alcohol. The scission could take place via the C₄-C₅ or the C₃-C₄ bonds. In both cases primary *C*-radicals would be generated, but the one resulting from the C₄-C₅ scission would be favored, through stabilization by the adjacent nitrogen and irreversible oxidation to an acyliminium ion (31). Since the scission does not affect the α-stereogenic center, the new 4-(oxo)homoalanine derivatives would be obtained as optically pure isomers such as compound (32).

The evolution of the iminium ion (31) under different conditions would lead to a variety of amino acids. The reduction of intermediate (31) would generate a *N-*methylated homoserine derivative (33), which can be transformed into other *N-*methylated amino acids. By contrast, an aqueous work-up would lead to the formation of the aldehyde (32), which could also be converted into many derivatives such as the α-allylglycine (34) or the diamino acid (35). The latter reaction would be very useful to introduce fluorescent groups for molecular imaging probes.

The conversion (30) → (32) is particularly interesting. Unlike the scission of serine or threonine, which generate a glycyl cation, the fragmentation of 4-hydroxyproline produces an iminium ion (31) whose α-position remains unaffected, and therefore the L-stereochemistry of the starting product is preserved. The intermediate (31) would be stabilized by formation of an *N,O-*acetal. This functional group is very versatile, and can be transformed into a variety of chemical functions: olefinic, alkyl or benzyl chains, alkyl phosphonates, etc. The generation of *N*-substituted amino acids such as compound (33) is important from a commercial standpoint, because these derivatives reach a high market value. With this method, *N*-substituted amino acids could be readily obtained from a relatively inexpensive precursor.

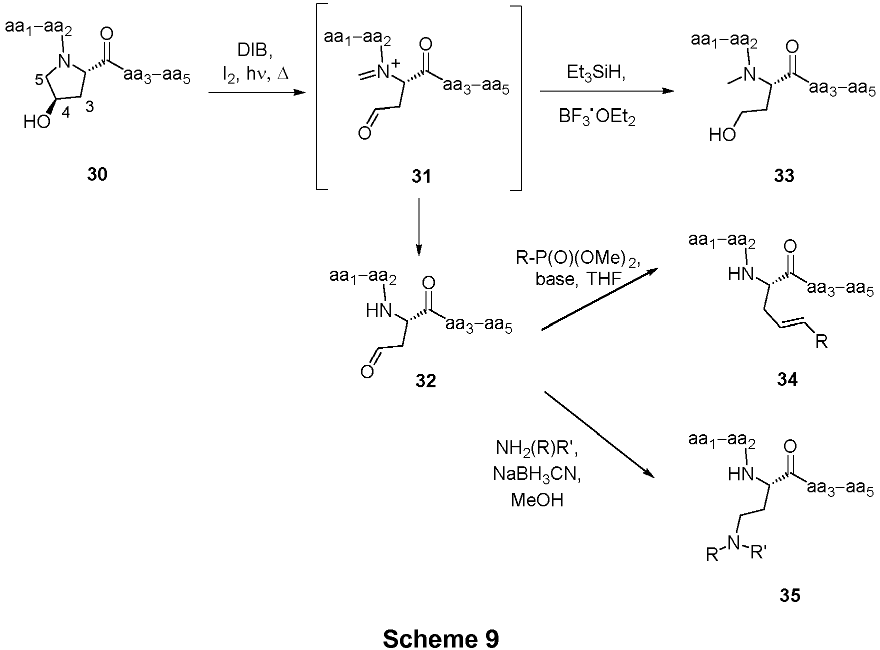

In summary, none of the methods described so far for the selective modification of amino acids and peptides has the double advantage of generating chiral amino acids and generating *N*-substituted products, hence the interest and innovation of the present invention.

The modification of hydroxyproline units in peptides would achieve several objectives:
1) Study of the influence of the amino acid hydrophilicity in the conformation and bioactivity of the peptide, since the procedure would allow the introduction of lateral chains with less polar substituents, such as alkyl or aryl, or with more polar groups, such as NH₂, OH, etc [Waldmann, H.; Janning, P. Chemical Biology, Chapters 3, 5 and 8, Wiley-VCH, Weinheim, 2004].
2) Introduction of lateral chains of different lengths with anchoring points for fluorophores or radioactive tracers, for studies of interactions with biological systems.
3) Transformation of cyclic amino acids into others with acyclic lateral chains. This change could affect the peptide conformation and therefore, allow the modulation of its biological or catalytic activity.

Therefore, an aspect of the present invention is a procedure for the selective modification of a hydroxylated cyclic amino acid with the formula (36), to generate a derivative with the formula (37), and conversion of the latter into amino acid derivatives with the formula (38), hereinafter called method of the invention, 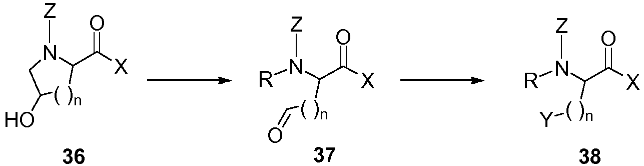 where
Z represents the amino acid *N*-substituent, and is selected from hydrogen, alkyl, acyl, carbamoyl, amino acyl, and peptidyl;
R represents one of the *N*-substituents of products (37) or (38) and is selected from H and alkyl;
n is an integer between 1 and 5;
X represents the carboxyl group substituent of the modified amino acid, and is selected from H, hydroxy, alkoxy, amino, aminoalkyl, amido, alkyl, acyl, aryl, amino acyl, and peptidyl;
Y represents groups of the lateral chain of the modified amino acid in compound (38), and is selected from carbonyl, carboxyl, CH₂OH, CH₂-amino, alkyl, acyl, CH₂-amino acyl, and CH₂-peptidyl, or alternatively is alkyl or acyl and is directly attached to a group X selected from O, NH or N-alkyl.

In a preferred embodiment for the method of invention, the conversion of the compound with the formula (36) into the product (37) is carried out using an radical scission where the configuration of the α-stereogenic center of the modified amino acid is retained, and therefore, the resulting products (37) are obtained with high optical purity.

In another embodiment for the method of invention, the conversion of the scission product (37) into the compound with the formula (38) is carried out using a reaction selected from, but not limited to, reductive amination, reduction-lactonization, or addition to the carbonyl group, including the Horner-Wadsworth-Emmons reaction.

In a particular aspect of the invention procedure, the amino acid derivative with the formula (37) is obtained by an oxidative radical scission reaction.

In a particular aspect of the invention procedure, the substrate with the formula (36) is a 4-hydroxyproline derivative where n = 1.

In a particular aspect of the method of invention, the amino acid derivative with the formula (36) presents Z selected from acyl or carbamoyl, and X selected from alkoxy or amino, and therefore product (37) is composed by one amino acid residue.

In another particular aspect of the method of invention, the amino acid derivative with the formula (36) presents Z or/and X selected from amino acyl or peptidyl, and therefore compound (37) is a peptide.

In a particular aspect of the method of invention, the oxidative radical scission of substrate (36) generates an acyliminium ion intermediate which is trapped by O-nucleophiles, to give products (37) where N-R is N-H when the *O-*nucleophile is water, or products (37) where N-R is a N,O-acetal when the *O-*nucleophile is an alcohol, alkoxylate, carboxylic acid or carboxylates.

In a particular embodiment of the method of invention, the oxidative radical scission step uses (diacetoxyiodo)benzene and iodine as reagents for the conversion of substrate (36) into product (37). For those skilled in the art, other reagents commonly used in radical scission reactions such as LTA, LTA-iodine, or HgO-iodine, can replace DIB-iodine in this step. Besides, experts in the field would recognize that a reductive radical scission can be used to give reduced derivatives of aldehyde (37).

In a preferred embodiment for the method of invention, the radical scission for the conversion of substrate (36) into a compound with the formula (37) is activated with an energy supply selected from, but not limited to, heating, irradiation with light or irradiation with microwaves.

In a more preferred embodiment, the conversion of substrate (36) into a compound with the formula (37) is carried out by an oxidative radical scission which uses visible light irradiation as the activation energy.

In a preferred embodiment for the method of invention, aprotic organic solvents are used, for example dichloromethane. For those skilled in the art, other organic solvents can be employed, for instance hexane, dichloroethane, toluene, acetonitrile, or ethyl acetate.

In other particular aspect of the method of invention, the scission product with the formula (37) where N-R is an *N,O*-acetal such as N-CH₂-OAc, is transformed into compounds (38) where the acetal is hydrolyzed and therefore R = H, or the acetal is reduced and therefore R = Me, or the acetal reacts with nucleophiles and therefore R = alkyl, alkylamino, etc.

Another aspect of the present invention is a compound with the formula (37), generated by radical scission of substrates with the formula (36).

In a particular embodiment of the present invention, the scission product (37) is selected from compounds with the formula (39-43).

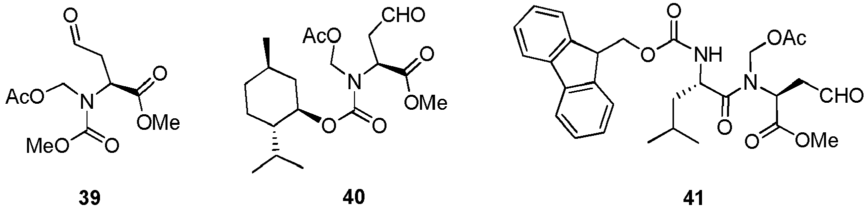

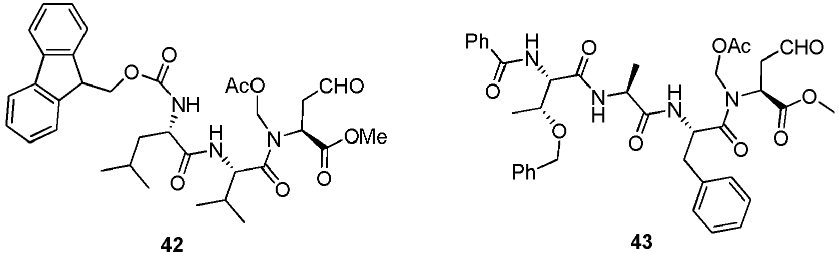

Another aspect of the present invention is a compound with the formula (38), obtained from a compound with formula (37), preferably by conversion of the carbonyl or/and N,O-acetal groups resulting from the radical scission, into other functional groups.

Thus, an aspect of the present invention is a compound with the formula (38), where Y is alkyl and is directly attached to a group X selected from O, NH or N-alkyl, and such lactone or lactam rings are formed by reduction or reductive amination of compounds with the formula (37).

In a particular embodiment of the present invention, the compound with formula (38) containing a lactone or lactam ring is selected from compounds of formula (44) to (49).

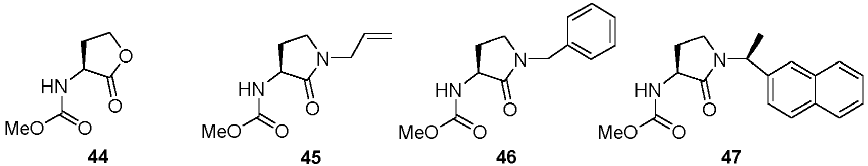

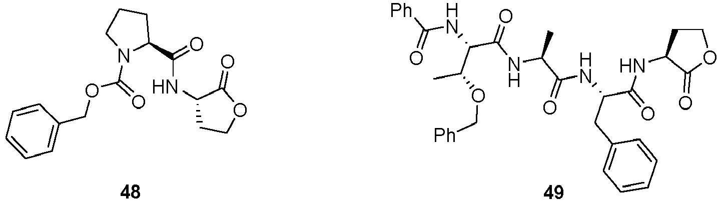

Another aspect of the present invention is a compound with the formula (38), where Y = methyleneamino, formed by reductive amination of compounds with the formula (37).

In a particular embodiment of the present invention, the compound of formula (38) with Y = methyleneamino is selected from compounds with the formula (50) to (57).

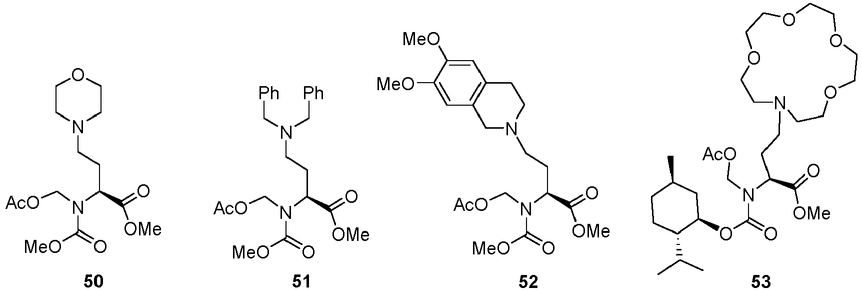

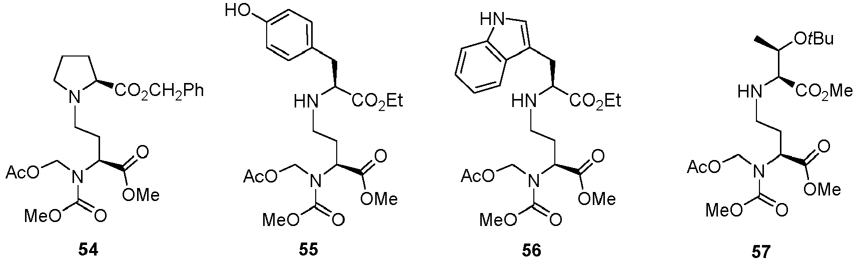

Another aspect of the present invention is a compound with the formula (38), where Y = alkyl or alkenyl, generated from compounds with the formula (37) by a Horner-Wadsworth-Emmons reaction (HWE), followed in some cases by reduction of the resulting olefin. For those skilled in the art, other related processes can also be used, such as the Wittig reaction, or other conventional methods, such as for example but not limited to, the addition or organometallic reagents to the aldehyde, followed by conversion of the resulting alcohol. For the skilled person, the alkenyl group generated in the HWE or related reactions can be transformed into other olefin using an olefin metathesis process.

In a particular embodiment of the present invention, the compound of formula (38) where Y = alkyl or alkenyl, is selected from compounds with the formula (58) to (60)

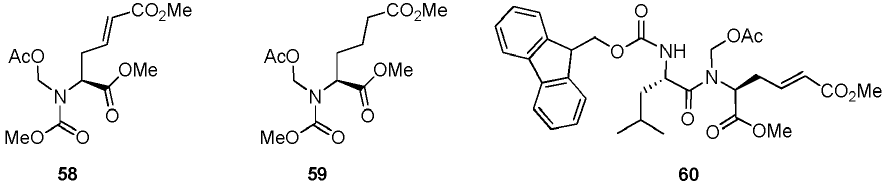

Another aspect of the present invention is a compound with the formula (38), where R = Me, formed by reduction of compounds (37) or (38) where N-R is a N,O-acetal, preferably NCH₂OAc.

In a particular embodiment of the present invention, the compound with the formula (38) with R = Me is selected from compounds with the formula (61) to (62).

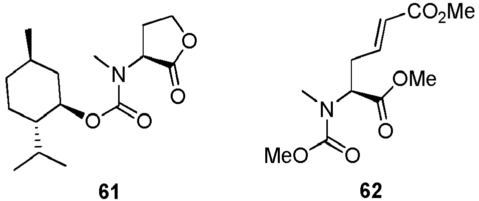

For persons skilled in the art, compounds with the formula (37) or (38) could be chemical precursors of other larger or more complex molecules.

Therefore, another aspect of the present invention is a compound with the formula (37) or (38) or its derivatives as previously described, for use thereof in the discovery of new catalytic or bioactive compounds and drugs. In effect, the libraries of such compounds would allow the determination of structure-activity relationships, and the identification of structural features that improve the potency, stability or bioavailability. These data would facilitate the design of second- or *n*-generation peptides with superior properties.

Another aspect of the present invention is a peptide with the formula (38) whose lateral chains present anchoring points for fluorophores or radioactive labels, so that these peptides could be used to develop new medical probes. For instance, those skilled in the art will infer that compounds (38) where Y = methyleneamino can be attached to the fluorophore reported by Imperiali [Loving, G.; Imperiali, B. J. Am. Chem. Soc., 2008, 130, 13630-13638], or to other fluorophores such as the dansyl, NBD or coumarin carbonyl groups, FIT, rhodamine, BODIPY, TAMRA, Cy5,5 and others [(a) Waldmann, H.; Janning, P. Chemical Biology, Wiley-VCH, Weinheim, 2004. (b) Loudet, A.; Burgess, K. Chem. Rev. 2007, 107, 4891-4932. (c) Katritzky, A. R.; Narindoshvili, T. Org. Biomol. Chem., 2009, 7, 627-634. (d) Lee, S.; Xie, J.; Chen, X. Chem. Rev. 2010, 110, 3087-3111. (e) Sinkeldam, R. W.; Greco, N. J.; Tor, Y. Chem. Rev. 2010, 110, 2579-2619]. It is also clear that compounds (38) with crown ethers, as exemplified by compound (53), can complex lanthanide metals and thus generate molecular imaging probes, for example for tumor detection. [(a) Dirscherl, G.; König, B. Eur. J. Org. Chem. 2008, 597-634. (b) Haas, K. L.; Franz, K. J. Chem. Rev. 2009, 109, 4921-4960. (c) Bünzli, C. G. C. Chem. Rev. 2010, 110, 2729-2755. (d) Glunde, K.; Artemov, D.; Penet, M. F.; Jacobs, M. A.; Bhujwalla, Z. M. Chem. Rev. 2010, 110, 3043-3059. (e) Yim, C. B.; van der Wildt, B.; Dijkgraaf, I.; Joosten, L.; Eek, A.; Versluis, C.; Rijkers, D. T. S.; Boerman, O. C.; Liskamp, R. M. J. ChemBioChem 2011, 12, 750-760].

Other additional aspect of the present invention is a compound with the formula (38) where N-R is a *N,O*-acetal group, preferably N-CH₂OAc, as previously described, for use thereof in the preparation of new compounds where N-R is N-alkyl, preferably N-methyl, by reduction or addition of nucleophiles.

Other additional aspect of the present invention is the use of a compound with the general formula (37) or (38), as previously described, for use thereof in the preparation of a larger peptide, a depsipeptide or a conjugate with other compounds including, but not limited to, lipids, carbohydrates and terpenes, according to conventional methodologies to prepare such conjugates.

### Examples of the invention

### Example 1: Scission-oxidation of simple hydroxyproline derivatives and application to the formation of optically pure α-aminolactones and α-aminolactams.

The conversion of simple hydroxyproline derivatives, namely compounds (36) where n = 1, is described below. Previous work in the area has been discussed in the State of the Art section. However, the scission of hydroxyproline derivatives such as methyl carbamate (62) (Scheme 10) had not been studied before.

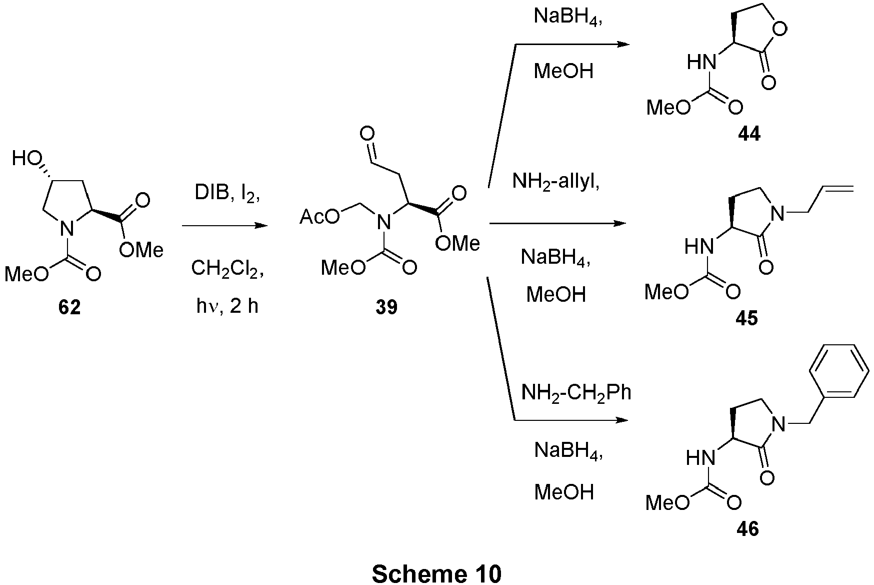

The scission was carried out by treatment of substrate (62) with (diacetoxyiodo)benzene and iodine, under irradiation with visible light. The process was completed in two hours at room temperature, affording the aldehyde (39) in good yields. The aldehyde (39) was then reduced to a homoserinol derivative, which underwent an intramolecular lactonization to give the high-profit lactone (44).

When aldehyde (39) was treated under reductive amination conditions, using primary amines such as allyl- or benzylamine, an intramolecular lactamization took place to give the lactams (45) and (46) with high optical purity.

In order to determine whether epimerization of the α-stereogenic center occurred during the process, the reductive amination of product (39) (Scheme 11) with the chiral amine (S) 1-(naphthyl)-ethylamine was then studied. The reductive amination was followed by lactamization, to give the lactam (47) as a single isomer.

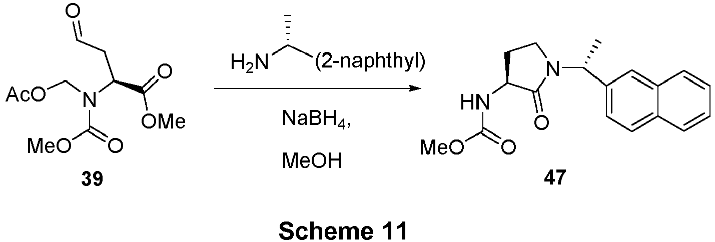

### Experimental procedure for example 1: Scission-Oxidation of hydroxyldroline derivatives and application to the formation of optically pure α-amino lactones and α-amino lactams.

*Me₂OC-Hyp-OMe* (62): Described in a) Barrett, A. G. M.; Pilipauskas, D. J. Org. Chem. 1991, 56, 2787-2800. b) Pickering, L.; Malhi, B. S.; Coe, P. L.; Walker, R. T. Tetrahedron 1995, 51, 2719-2728. c) Shono, T.; Matsumura, Y.; Tsubata, K.; Sugihara, Y.; Yamane, S.; Kanazawa, T.; Aoki, T. J. Am. Chem. Soc. 1982, 104, 6697-6703.

*(2S)-N-(Acetoxymethyl)-N-methoxycarbonyl- 2-(2-oxoethyl)glycine methyl ester* (39): To a solution of 4R-hydroxy-L-proline methyl carbamate (62) (500 mg, 2.46 mmol) in dry dichloromethane (20 mL) was added (diacetoxyiodo)benzene (1584 mg, 4.92 mmol) and iodine (312 mg, 1.23 mmol). The mixture was stirred at room temperature for 2.5 h, under irradiation with visible light (80-W tungsten-filament lamp); then was poured into 10% aqueous sodium thiosulfate, and extracted with dichloromethane. The residue was purified by column chromatography on silica gel (hexane/EtOAc, 80:20), yielding the scission product (39) (510 mg, 79%), as a yellowish oil: [α]_{D} = -84.5 (c 0.35, CHCl₃); IR (CHCl₃) vₘₐₓ. 1728, 1480, 1441 cm⁻¹; ¹H NMR (500 MHz, CDCl₃, 70 °C) *δ*_{H} 2.04 (3H, s, Ac), 3.02 (1 H, m, CHₐCHO), 3.30 (1 H, dd, ***J*** = 5.7, 18.3 Hz, CH_{b}CHO), 3.72 (3H, s, OMe), 3.75 (3H, s, OMe), 4.90 (1 H, dd, ***J*** = 5.7, 5.7 Hz, CHN), 5.43 (2H, s, OCH₂N), 9.75 (1 H, s, CHO); ¹³C NMR (125.7 MHz, CDCl₃, 70 °C) *δ*_{C} 20.7 (CH₃), 44.7 (CH₂), 52.7 (CH₃), 53.4 (CH₃), 55.6 (CH), 72.5 (CH₂), 155.6 (C), 170.2 (C), 170.6 (C), 198.0 (C); MS (EI) m/z (relative intensity) 218 (M⁺-CH₂CHO, 7), 202 (M⁺ - CO₂Me, 9), 158 (M⁺ - CH₂CHO - AcOH, 36), 142 ([CH₂=N-CH(CO₂Me)CH₂CHO - H]⁺, 100), 129 ([N-CH(CO₂Me)CH₂CHO + H]⁺, 54). HRMS calcd for C₈H₁₂NO₆, 218.0665, found 218.0657; calculated for C₈H₁₂NO₅, 202.0715, found 202.0709; calculated for C₆H₈NO₄, 158.0453, found 158.0450; calculated for C₆H₈NO₃, 142.0504, found 142.0507. Elemental analysis: Calculated for C₁₀H₁₅NO₇: C, 45.98; H, 5.79; N, 5.36; found C, 45.67; H, 5.91; N, 5.46.

*N-(Methoxycarbonyl)homoserine lactone* (44): A solution of aldehyde (39) (53 mg, 0.20 mmol) in dry methanol (3 mL) was treated with sodium borohydride (10 mg, 0.26 mmol). After stirring for 4 h at room temperature, the reaction mixture was poured into water and extracted with EtOAc. The organic layer was dried over sodium thiosulfate, filtered and evaporated under vacuum. The residue was purified by rotatory chromatography (hexane/EtOAc, 60:40), affording the lactone (44) (25 mg, 77%), as a white solid: ¹H NMR (500 MHz, CDCl₃, 26 °C) *δ*_{H} 2.22 (1 H, m, 3-Hₐ), 2.74 (1 H, m, 3-H_{b}), 3.70 (3H, s, OMe), 4.25 (1 H, m, 2-H), 4.20-4.30 (2H, m, 4-H₂), 5.44 (1 H, br b, NH); ¹³C NMR (125.7 MHz, CDCl₃, 26 °C) *δ*_{C} 30.3 (CH₂), 50.5 (CH), 52.6 (CH₃), 65.7 (CH₂), 156.7 (C), 175.0 (C); MS (EI) m/z (relative intensity) 159 (M⁺, 5), 128 (M⁺ - OMe, 16), 115 (M⁺ - CO₂, 100),100 (M⁺ - CO₂Me, 60). HRMS calcd for C₆H₉NO₄, 159.0532, found 159.0531; calculated for C₅H₆NO₃, 128.0348, found 128.0349; calculated for C₅H₉NO₂, 115.0633, found 115.0634; calculated for C₄H₆NO₂, 100.0399, found 100.0402. Elemental analysis: Calculated for C₆H₉NO₄: C, 45.28; H, 5.70; N, 8.80; found C, 45.13; H, 5.80; N, 8.56.

*(S) 1-Allyl-3-(N-methoxycarbonyl)amino-2-pyrrolidinone* (45): A solution of the aldehyde (39) (53 mg, 0.20 mmol) in dry methanol (3 mL) was treated with allylamine (20 µL, 0.27 mmol) and triethylamine (38 µL, 0.27 mmol), and stirred for 1 h at room temperature (26 °C). Then sodium borohydride (10 mg, 0.26 mmol) was added, and the mixture was heated to 45°C for 20 h, then was allowed to cool to room temperature, was poured into water and extracted with EtOAc. The organic layer was dried and concentrated in the usual way, and the residue was purified by rotatory chromatography (hexane/EtOAc, 40:60), yielding the lactam (45) (31 mg, 77 %) as a white solid: m.p. 92-94 °C; [α]_{D} = -9.16 (*c* 0.3, CHCl₃); IR (CHCl₃) vₘₐₓ. 3426, 1722, 1695, 1513 cm⁻¹; ¹H NMR (500 MHz, CDCl₃, 26 °C) *δ*_{H} 1.89 (1H, m, 3-Hₐ), 2.61 (1H, m, 3-H_{b}), 3.25-3.35 (2H, m, 4-H₂), 3.67 (3H, s, OMe), 3.86 (1H, dd, ***J*** = 6.2, 15.2 Hz, NCHₐ-vinil), 3.93 (1 H, dd, ***J*** = 6.0, 15.2 Hz, NCH_{b}-vinil), 4.22 (1 H, dd, ***J*** = 9.2, 9.3 Hz, 2-H), 5.18 (1 H, dddd, ***J =*** 1.3, 1.3, 1.4, 8.8 Hz, 3'-Hₐ), 5.20-5.22 (1H, m, 3'-H_{b}), 5.70 (1 H, m, 2'-H); ¹³C NMR (125.7 MHz, CDCl₃, 26 °C) *δ*_{C} 27.7 (CH₂), 43.6 (CH₂), 45.8 (CH₂), 52.2 (CH₃), 52.8 (CH), 118.4 (CH₂), 131.6 (CH), 157.0 (C), 171.8 (C); MS (EI) m/z (relative intensity) 198 (M⁺, 33), 123 (M⁺ - NH₂CO₂Me, 100). HRMS calcd for C₉H₁₄N₂O₃, 198.1004, found 198.1011; calculated for C₈H₁₁N₂O₂, 167.0821, found 167.0822; calculated for C₇H₉NO, 123.0684, found 123.0682. Elemental analysis: Calculated for C₉H₁₄N₂O₃: C, 54.53; H, 7.12; N, 14.13; found C, 54.35; H, 7.20; N, 14.36.

*(S) 1-Benzyl-3-(N-methoxycarbonyl)amino-2-pyrrolidinone* (46): A solution of aldehyde (39) (53 mg, 0.20 mmol) in dry methanol (3 mL) was treated with benzylamine (30 µL, 0.28 mmol) and triethylamine (38 µL, 0.27 mmol) and stirred for 1 h at room temperature (26 °C). Then sodium borohydride (10 mg, 0.26 mmol) was added, and the mixture was heated to 45°C for 20 h. Then it was allowed to cool to room temperature, poured into water and extracted with EtOAc. The organic layer was dried and concentrated as usual, and the residue was purified by rotatory chromatography (hexane/EtOAc, 50:50), affording the product (46) (39 mg, 77%), as a white solid: m.p. 121-123 °C; [α]_{D} = -7.96 (c 0.33, CHCl₃); IR (CHCl₃) vₘₐₓ. 1722, 1697, 1510 cm⁻¹ ; ¹H NMR (500 MHz, CDCl₃ with TMS, 26 °C) *δ*_{H} 1.86 (1 H, m, 4-Hₐ), 2.61 (1 H, m, 4-H_{b}), 3.18-3.25 (2H, m, 5-H₂), 3.70 (3H, s, OMe), 4.26 (1 H, m, 3-H), 4.46 (1 H, d, *J =* 15 Hz, OCHₐPh), 4.51 (1 H, d, *J* = 14.7 Hz, OCH_{b}Ph), 5.45 (1 H, br b, NH), 7.23 (2H, br d, *J* = 7.8 Hz, Ar), 7.27-7.36 (8H, m, Ar); ¹³C NMR (125.7 MHz, CDCl₃, 26 °C) *δ*_{C} 27.7 (CH₂), 43.3 (CH₂), 47.1 (CH₂), 52.2 (CH₃), 52.8 (CH), 127.7 (CH), 128.0 (2 x CH), 128.7 (2 x CH), 135.7 (C), 157.0 (C), 171.9 (C); MS (EI) m/z (relative intensity) 248 (M⁺, 34), 173 (M⁺ - NH₂CO₂Me, 54), 91 ([OCH₂Ph]⁺, 100). HRMS calcd for C₁₃H₁₆N₂O₃, 248.1161, found 248.1162; calculated for C₁₁H₁₁NO, 173.0841, found 173.0846. Elemental analysis: Calculated for C₁₃H₁₆N₂O₃: C, 62.89; H, 6.50; N, 11.28; found C, 63.11; H, 6.75; N, 10.95.

*(S) 1-((S)-1-(2-Naphthalenyl)ethyl)-3-(N-methoxycarbonyl)amino-2-pyrrolidinone* (47): A solution of the aldehyde (39) (53 mg, 0.20 mmol) in dry methanol (3 mL) was treated with (*R*)-(+)-1-(2-naphthyl)ethylamine (48 mg, 0.28 mmol) and triethylamine (38 µL, 0.27 mmol). After stirring for 1 h at 26 °C, sodium borohydride (10 mg, 0.26 mmol) was added, and the mixture was heated to 45 °C for 20 h. Then it was allowed to cool to 26 °C, poured into water and extracted with EtOAc. The organic layer was dried and concentrated as usual, and the residue was purified by rotatory chromatography (hexane/EtOAc 50:50), yielding the lactam (47) (42 mg, 66%) as a white solid; m.p. 148-150 °C; [α]_{D} = +217.9 (*c* 0.36, CHCl₃); IR (CHCl₃) vₘₐₓ. 3426, 3018, 1721, 1688, 1511 cm⁻¹; ¹H NMR (500 MHz, CDCl₃, 26 °C) *δ*_{H} 1.66 (3H, d, ***J*** = 7.1 Hz, Me), 1.85 (1 H, m, 4-Hₐ), 2.52 (1 H, m, 4-H_{b}), 2.85 (1 H, ddd, *J* = 6.8, 6.8, 9.8 Hz, 5-Hₐ), 3.32 (1 H, dd, ***J*** = 9.3, 9.7 Hz, 5-H_{b}), 3.69 (3H, s, OMe), 4.22 (1 H, m, 3-H), 5.56 (1 H, b.a., NH), 5.64 (1 H, ddd, ***J*** = 7.0, 7.0, 7.1 Hz, CH(Me)Ar), 7.35 (1 H, dd, ***J*** = 1.5, 8.6 Hz, Ar), 7.45-7.52 (2H, m, Ar), 7.72 (1 H, s, Ar), 7.78-7.88 (3H, m, Ar); ¹³C NMR (125.7 MHz, CDCl₃, 26 °C) *δ*_{C} 15.9 (CH₃), 27.7 (CH₂), 39.4 (CH₂), 50.0 (CH), 52.2 (CH₃), 53.2 (CH), 125.5 (2 x CH), 126.1 (CH), 126.3 (CH), 127.6 (CH), 127.9 (CH), 128.5 (CH), 132.8 (C), 133.1 (C), 136.9 (C), 157.0 (C), 171.6 (C); MS (EI) m/z (relative intensity) 312 (M⁺, 10), 280 (M⁺ - MeOH, 20), 155 ([Me-CH-naphthyl]⁺, 100). HRMS calcd for C₁₈H₂₀N₂O₃, 312.1474, found 312.1472; calculated for C₁₂H₁₁, 155.0861, found 155.0859. Elemental analysis: Calculated for C₁₈H₂₀N₂O₃: C, 69.21; H, 6.45; N, 8.97; found C, 68.96; H, 6.42; N, 8.79.

### Example 2: Reductive amination of the aldehydes resulting from the oxidative radical scission of simple hydroxyproline derivatives.

When the reductive amination was carried out with secondary amines, such as morpholin, dibenzylamine or 6,7-dimethoxy-1,2,3,4-tetrahydroisoquinoline, the 4-amino homoalanine derivatives (50)-(52) were obtained in high yields (Scheme 12).

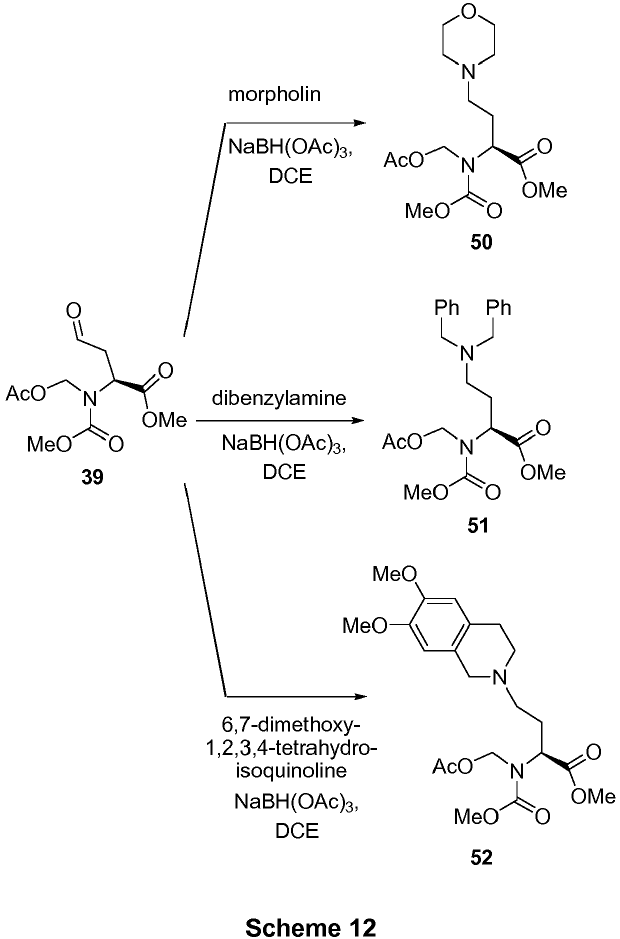

In order to determine whether epimerization of the α-stereogenic center took place during the process, the reaction was repeated with substrates possessing chiral auxiliaries, such as the (-)-menthyloxycarbonyl group. Thus, the menthyl derivative (63) (Scheme 13) was prepared and treated under the oxidative radical scission conditions, affording exclusively the aldehyde (40), thus confirming that the scission proceeded with negligible epimerization. However, isomerization does take place slowly when the aldehyde is in solution, and for instance, a 20% epimerization was observed in solutions of chloroform with traces of acid after 15 days.

The aldehyde (40) underwent a reductive amination with the crown ether 1-azo-15-crown-5, giving the unnatural amino acid (53) as a pure diastereomer. The amino acids with crown ether substituents are of great academic and commercial interest, as components of artificial ion channels or as chelators i.e. in complexes with lanthanides or other metals for molecular imaging (medical probes), and therefore this result is particularly relevant.

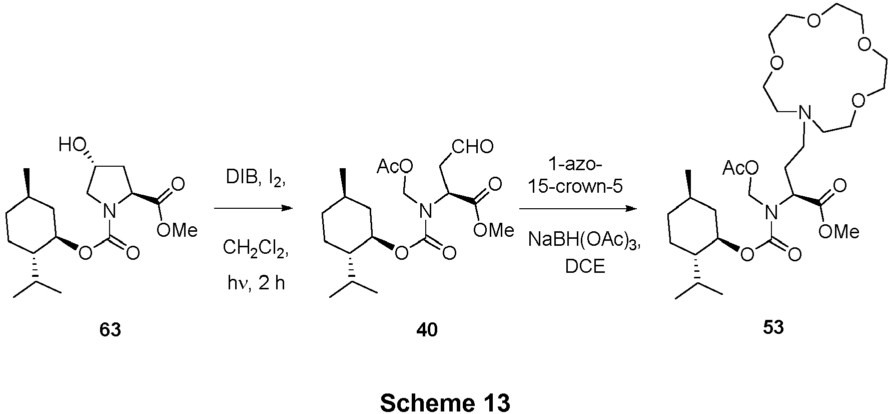

### Experimental procedure for example 2: Reductive amination reaction with secondary amines, to give 4-amino homoalanine derivatives.

*(S) N-Acetoxymethyl-N-methoxycarbonyl-4-(morpholino)homoalanine methyl ester* (50): A solution of the aldehyde (39) (53 mg, 0.20 mmol) in dry dichloromethane (3 mL) was treated with morpholine (23 µL, 0.26 mmol) and triethylamine (38 µL, 0.27 mmol) for 10 min. at 26 °C. Then sodium (triacetoxy)borohydride (69 mg, 0.32 mmol) was added, and the mixture was stirred for other 2.5 h. The solution was poured into saturated aqueous NaHCO₃, and extracted with dichloromethane. The organic layer was washed with brine and then dried and concentrated as usual. The residue was purified by rotatory chromatography (hexane/EtOAc, 60:40), yielding the product (50) (59 mg, 88%), as a yellowish slurry: [α]_{D} = -35.1 (c 0.34, CHCl₃); IR (CHCl₃) vₘₐₓ. 1740, 1717, 1447 cm⁻¹; ¹H NMR (500 MHz, CDCl₃, 26 °C) 2:1 rotamer mixture *δ*_{H} 2.04 (3H, s, Me), 2.03 (1H, m, 3-Hₐ), 2.52 (1H, m, 3-H_{b}), 2.30-2.55 (6H, m, 4-H₂ + 1'-H₂), 3.70 (3H, s, OMe), 3.72/3.75 (3H, s/s, OMe), 3.65-3.75 (4H, m, 2'-H₄), 4.59 (1 H, m, 2-H), 5.35-5.51 (2H, m, OCH₂N); ¹³C NMR (125.7 MHz, CDCl₃, 26 °C) *δ*_{C} 20.9 (CH₃), 26.3/27.0 (CH₂), 52.4 (CH₃), 53.5 (CH₃ + 2 x CH₂), 54.6/54.8 (CH₂), 58.2/58.3 (CH), 66.7 (2 x CH₂), 71.5/72.2 (CH₂), 155.8/156.4 (C), 170.7 (C), 171.5 (C); MS (EI) m/z (relative intensity) 332 (M⁺, 4), 273 (M⁺ - CO₂Me, 4), 100 ([CH₂CH₂CHCO₂Me]⁺, 100). HRMS calcd for C₁₄H₂₄N₂O₇, 332.1584, found 332.1575; calculated for C₁₂H₂₁N₂O₅, 273.1450, found 273.1464. Elemental analysis:Calculated for C₁₄H₂₄N₂O₇: C, 50.59; H, 7.28; N, 8.43; found C, 50.53; H, 7.26; N, 8.44.

*(S) N-Acetoxymethyl-N-methoxycarbonyl-4-(dibenzylamino)homoalanine methyl ester* (51): A solution of the aldehyde (39) (53 mg, 0.20 mmol) in dry dichloromethane (3 mL) was treated with dibenzylamine (50 µL, 0.26 mmol) and triethylamine (38 µL, 0.27 mmol) for 10 min. at 26 °C. Then sodium (triacetoxy)borohydride (69 mg, 0.32 mmol) was added and the mixture was stirred for other 4 h. After usual work-up and concentration, the residue was purified by rotatory chromatography (hexane/EtOAc, 95:5), yielding the product (51) (68 mg, 76%), as a yellowish oil: [α]_{D} = -52.02 (c 0.33, CHCl₃); IR (CHCl₃) vₘₐₓ. 1740, 1716, 1479, 1450 cm⁻¹; ¹H NMR (500 MHz, CDCl₃, 26 °C) 3:2 rotamer mixture *δ*_{H} 1.94 (3H, s, Ac), 1.95 (1 H, m, 3-Hₐ), 2.30 (1 H, m, 3-H_{b}), 2.50 (1 H, m, 4-Hₐ), 2.55 (1 H, m, 4-H_{b}), 3.50 (2H, m, 2 x OCHₐPh), 3.61 (3H, s, OMe), 3.62 (2H, m, 2 x OCH_{b}Ph), 3.66 (3H, s, OMe), 4.66 (1 H, m, 2-H), 5.06/5.14 (1 H, d, *J* = 10.9 Hz/1H, d, *J* = 10.6 Hz), 5.21 (1H, d, *J* = 11.3 Hz/ 1H, d, *J* = 11.0 Hz), 7.22-7.38 (10H, m, Ar); ¹³C NMR (125.7 MHz, CDCl₃, 26 °C) *δ*_{C} 20.8 (CH₃), 27.2/28.1 (CH₂), 49.7 (CH₂), 52.3 (CH₃), 53.3 (CH₃), 57.6/57.9 (CH), 58.4 (2 x CH₂), 71.1/71.6 (CH₂), 127.0 (2 x CH₂), 128.3 (4 x CH), 129.0 (4 x CH), 139.2 (2 x C), 155.9 (C), 170.6 (C), 171.7 (C); MS (EI) m/z (relative intensity) 442 (M⁺, 1), 383 (M⁺ - CO₂Me, 3), 291 (M⁺ - AcOH - CH₂Ph, 9), 210 ([(PhCH₂)₂N=CH₂]⁺, 95), 91 ([(PhCH₂]⁺, 100). HRMS calcd for C₂₄H₃₀N₂O₆, 442.2104, found 442.2119; calculated for C₂₂H₂₇N₂O₄, 383.1971, found 383.1975; calculated for C₁₅H₁₉N₂O₄, 291.1345, found 291.1359; calculated for C₁₅H₁₆N, 210.1283, found 210.1292; calculated for C₇H₇, 91.0548, found 91.0547. Elemental analysis: Calculated for C₂₄H₃₀N₂O₆: C, 65.14; H, 6.83; N, 6.33; found C, 65.16; H, 6.96; N, 6.31.

*(S) N-Acetoxymethyl-N-methoxycarbonyl-4-(6,7-dimethoxy-1,2,3,4-tetrahydro-2-isoquinolinyl)homoalanine methyl ester* (52): A solution of the aldehyde (39) (53 mg, 0.20 mmol) in dry dichloroethane (3 mL) was treated with 6,7-dimethoxy-1,2,3,4-tetrahydroisoquinoline hydrochloride (61 mg, 0.26 mmol) and triethylamine (38 µL, 0.27 mmol) and stirred for 10 min. at 26 °C. Then sodium (triacetoxy)borohydride (69 mg, 0.32 mmol) was added and the stirring continued for 5 h. After usual work-up and evaporation, the residue was purified by rotatory chromatography (hexane/EtOAc, 50:50), affording product (52) (64 mg, 72%), as a yellowish slurry: [α]_{D} = -36.03 (c 0.34, CHCl₃); IR (CHCl₃) vₘₐₓ. 1739, 1717, 1518 cm⁻¹; ¹H NMR (500 MHz, CDCl₃, 26 °C) 2:1 rotamer mixture *δ*_{H} 2.03 (3H, s, Ac), 2.09 (1 H, m, 3-Hₐ), 2.36 (1 H, m, 3-H_{b}), 2.57 (1 H, m, 4-H₂), 2.68 (1 H, m, 1'-Hₐ), 2.74 (1 H, m, 1'-H_{b}), 2.80 (2H, dd, J = 5.6, 5.7, 2'-H2), 3.51 (1 H, brd, J = 13.3 Hz), 3.60 (1 H, d, J = 14.4 Hz), 3.68 (3H, s, OMe), 3.76/3.71 (3H, s, OMe), 3.82 (3H, s, OMe), 3.83 (3H, s, OMe), 4.62 (1 H, m, 2-H), 5.36/5.33 (1 H, [d, *J =* 11.3 Hz/ d, *J* = 11 Hz], NCHₐO), 5.48/5.44 (1 H, [d, *J* = 11.2 Hz/ d, *J* = 11 Hz], NCH_{b}O), 6.50 (1 H, s, Ar), 6.58 (1 H, s, Ar); ¹³C NMR (125.7 MHz, CDCl₃, 26 °C) *δ*_{C} 20.9 (CH₃), 27.0/27.8 (CH₂), 28.5 (CH₂), 50.8 (CH₂), 52.3 (CH₃), 53.4 (CH₃), 54.0/54.2 (CH), 55.4 (CH₂), 55.8 (CH₃), 58.2/58.6 (CH), 71.7/72.3 (CH₂), 109.4 (CH), 111.3 (CH), 126.1 (2 x C), 147.2 (C), 147.5 (C), 155.8/156.2 (C), 170.6/170.8 (C), 171.6 (C); MS (EI) m/z (relative intensity) 438 (M⁺, 3), 379 (M⁺-CO₂Me, 7), 206 ([2-methylen-6,7-dimethoxy-1,2,3,4-tetrahydroisoquinoline]⁺, 90), 210 ([6,7-dimethoxy-1,2,3,4-tetrahydroisoquinoline -H]⁺, 100). HRMS calcd for C₂₁H₃₀N₂O₈, 438.2002, found 438.2017; calculated for C₁₉H₂₇N₂O₆, 379.1869, found 379.1854; calculated for C₁₂H₁₆NO₂, 206.1181, found 206.1171. Elemental analysis: Calculated for C₂₁H₃₀N₂O₈: C, 57.52; H, 6.90; N, 6.39; found C, 57.62; H, 6.96; N, 6.42.

*N-[(1R,2S,5R)-Menthyloxycarbonyl]-4R-hydroxy-L-proline methyl ester* (63): To 4R-hydroxy-L-proline methyl ester hydrochloride (600 mg, 3.3 mmol) was added a saturated aqueous NaHCO₃ solution (10 mL). After cooling to 0 °C, a solution of (-)-menthyl chloroformate (0.85 mL, 3.96 mmol) in THF (10 mL) was added and the mixture was allowed to reach room temperature for 3 h. Then it was poured into water and extracted with EtOAc. The organic layer was dried, filtered and concentrated as usual, yielding menthyl carbamate (63) (1014 mg, 94%) as an oil. [α]_{D} =-99.27 (*c* 0.37, CHCl₃); IR (CHCl₃) vₘₐₓ. 3417, 1746, 1694, 1420 cm⁻¹; ¹H NMR (500 MHz, CDCl₃, 70 °C) *δ*_{H} 0.81 (3H, d, *J* = 7.0 Hz, Me), 0.80-0.91 (2H, m, 4'-Hₐ + 6'-Hₐ), 0.89 (3H, d, *J* = 5.7 Hz, Me), 1.07 (1 H, m, 3'-Hₐ), 1.33 (1 H, m, 2'-H), 1.46 (1 H, m, 5'-H), 1.64-1.73 (2H, m, 3'-H_{b} + 4'-H_{b}), 1.85 (1 H, br b, OH), 1.93 (1 H, m, 2"-H), 2.04 (1 H, m, 6'-H_{b}), 2.11 (1 H, ddd, *J* = 5.1, 7.0, 13.3 Hz, 3-H_{b}), 2.27 (1 H, m, 3-Hₐ), 3.52 (1 H, m, 5-Hₐ), 3.67 (1 H, dd, *J* = 4.7, 11.7 Hz, 5-H_{b}), 3.71 (3H, s, OMe), 4.44 (1H, m, 2-H), 4.48 (1H, m, 4-H), 4.55 (1 H, ddd, *J* = 4.4, 10.7, 11.0 Hz, 1'-H); ¹³C NMR (125.7 MHz, CDCl₃, 70 °C) *δ*_{C} 16.9 (CH₃), 20.7 (CH₃), 21.9 (CH₃), 24.2 (CH₂), 26.8 (CH), 31.5 (CH), 34.5 (CH₂), 38.8 (CH₂), 41.7 (CH₂), 47.7 (CH), 52.0 (CH₃), 54.8 (CH₂), 57.9 (CH), 69.4 (CH), 75.9 (CH), 154.2 (C), 173.1 (C); MS (EI) m/z (relative intensity) 327 (M⁺, <1), 268 (M⁺ - CO₂Me, 6), 138 ([menthyl - H]⁺, 31), 95 ([menthyl - CHMe₂ - H]⁺, 100), 81 ([cyclohexenyl]⁺, 89). HRMS calcd for C₁₇H₂₉NO₅, 327.2046, found 327.2053; calculated for C₁₅H₂₆NO₃, 268.1913, found 268.1908; calculated for C₁₀H₁₈ 138.1409, found 138.1410; calculated for C₇H₁₁ 95.0861, found 95.0864; calculated for C₆H₉ 81.0704, found 81.0704. Elemental analysis: Calculated for C₁₇H₂₉NO₅: C, 62.36; H, 8.93; N, 4.28; found C, 62.30; H, 8.80; N, 4.35.

(2S) *N-(Acetoxymethyl)-N-[(1R,2S,5R)-menthyloxycarbonyl]-2-(2-oxoethyl)glycine methyl ester* (40): A solution of the carbamate (63) (425 mg, 1.29 mmol) in dry dichloromethane (15 mL) was treated with (diacetoxyiodo)benzene (836 mg, 2.59 mmol) and iodine (164 mg, 0.64 mmol). The mixture was stirred at room temperature for 2.5 h, under irradiation with visible light (80 W-tungsten-filament lamp). Then was poured into 10% aqueous Na₂S₂O₃ and extracted with dichloromethane. The organic layer was dried, filtered and concentrated as usual, and the residue was purified by column chromatography on silica gel (hexane/EtOAc, 85:15), yielding the scission product (40) (401 mg, 80%) as a yellowish oil: [α]_{D} = -95.7 (c 0.87, CHCl₃); IR (CHCl₃) vₘₐₓ. 1741, 1707, 1458, 1420 cm⁻¹; ¹H NMR (500 MHz, CDCl₃, 70 °C) *δ*_{H} 0.78 (3H, d, *J* = 6.9 Hz, Me), 0.86 (1 H, m, 4'-Hₐ), 0.89 (3H, d, *J* = 7.3 Hz, Me), 0.91 (3H, d, *J* = 6.7 Hz, Me), 0.98-1.10 (2H, m, 3'-Hₐ + 6'-Hₐ), 1.37 (1 H, m, 2'-H), 1.47 (1 H, m, 5'-H), 1.64-1.72 (2H, m, 3'-H_{b} + 4'-H_{b}), 1.83 (1 H, m, 2"-H), 2.02 (3H, s, Ac), 2.03 (1 H, m, 6'-H_{b}), 2.95 (1 H, m, 3-Hₐ), 3.31 (1 H, dd, *J* = 6.0, 17.9 Hz, 3-H_{b}), 3.70 (3H, s, OMe), 4.63 (1 H, br ddd, *J* = 4.1, 10.7, 10.8 Hz, 1'-H), 4.86 (1 H, dd, *J* = 6.6, 6.7 Hz, 2-H), 5.40 (1 H, d, *J* = 11.1 Hz, OCHₐN), 5.43 (1 H, brd, *J* = 11.4 Hz, OCH_{b}N), 9.75 (1 H, s, CHO); ¹³C NMR (125.7 MHz, CDCl₃, 70 °C) *δ*_{C} 16.3 (CH₃), 20.5 (CH₃), 20.6 (CH₃), 21.8 (CH₃), 23.7 (CH₂), 26.4 (CH), 31.4 (CH), 34.2 (CH₂), 41.1 (CH₂), 44.8 (CH₂), 47.4 (CH), 52.4 (CH₃), 55.5 (CH), 72.4 (CH₂), 77.2 (CH), 154.8 (C), 170.2 (C), 170.4 (C), 197.9 (C); MS (EI) m/z (relative intensity) 370 (M⁺ - Me, <1), 144 (CH₂=N-CH(CO₂Me)CH₂CHO + H, 34), 83 ([H(O)C-CH₂-CH-CO₂Me - MeOH]⁺, 100). HRMS calcd for C₁₈H₂₈NO₇, 370.1866, found 370.1861; calculated for C₆H₁₀NO₃, 144.0661, found 144.0666; calculated for C₄H₃O₂, 83.0133, found 83.0136. Elemental analysis: Calculated for C₁₉H₃₁NO₇: C, 59.20; H, 8.11; N, 3.63; found C, 59.21; H, 8.16; N, 3.70.

*(S) N-(Acetoxymethyl)-N-[(1R,2S,5R)-menthyloxycarbonyl]-4-(1,4,7,10-tetraoxa-13-azacyclopentadecan-13-yl)homoalanine methyl ester* (53): A solution of the aldehyde (40) (78 mg, 0.20 mmol) in dry dichloroethane (3 mL) was treated with 1-aza-15-crown-5 (58 mg, 0.26 mmol) and triethylamine (38 µL, 0.27 mmol) and stirred for 10 min. at 26 °C. Then sodium (triacetoxy)borohydride (69 mg, 0.32 mmol) was added and the stirring continued for 2.5 h. After usual work-up and concentration, the residue was purified by rotatory chromatography (hexane/EtOAc, 98:2), yielding the product (53) (108 mg, 91%) as a yellowish slurry: [α]_{D} =-51.22 (c 0.41, CHCl₃); ¹H NMR (500 MHz, CDCl₃, 70 °C) *δ*_{H} 0.77 (3H, d, *J* = 6.9 Hz, Me), 0.80-0.90 (1 H, m, 4'-Hₐ), 0.88 (3H, d, J = 7.3 Hz, Me), 0.89 (3H, d, *J = 6.6* Hz, Me), 0.96 (1 H, m, 6'-H_{b}), 1.05 (1 H, m, 3'-Hₐ), 1.36 (1 H, m, 2'-H), 1.55 (1 H, m, 5'-H), 1.62-1.69 (2H, m, 4'-H_{b} + 3'-H_{b}), 1.86 (1 H, m, 2"-H), 1.96 (1 H, m, 3-Hₐ), 2.00 (3H, s, Ac), 2.02 (1 H, m, 6'-Hₐ), 2.23 (1 H, m, 3-H_{b}), 2.57-2.69 (2H, m, 4-H₂), 2.72-2.82 (4H, m, 2 x CH₂N), 3.58-3.64 (16H, m, 8 x CH₂O), 3.66 (3H, s, OMe), 4.52 (1 H, br b, 2-H), 4.60 (1 H, ddd, *J* = 4.4, 10.8, 11.0 Hz, 1'-H), 5.35 (1 H, br d, *J* = 11.0 Hz, OCHₐN), 5.45 (1 H, d, *J* = 11.0 Hz, OCH_{b}N); ¹³C NMR (125.7 MHz, CDCl₃, 70 °C) *δ*_{C} 16.3 (CH₃), 20.7 (CH₃), 21.8 (CH₃), 23.6 (CH₂), 25.9 (CH), 28.0 (CH₂), 31.4 (CH), 34.3 (CH₂), 41.1 (CH₂), 47.4 (CH), 51.9 (CH₃), 53.0 (CH₂), 54.7 (2 x CH₂), 58.3 (CH), 69.8 (2 x CH₂), 70.3 (2 x CH₂), 70.6 (2 x CH₂), 71.1 (2 x CH₂), 76.7 (CH), 155.3 (C), 170.3 (C), 171.7 (C); MS (EI) m/z (relative intensity) 588 (M⁺, 1), 545 (M⁺ - CHMe₂, 2), 529 (M⁺ - OAc, 4), 232 ([1-methylen-1-aza-15-crown-5]⁺, 100. HRMS calcd for C₂₉H₅₂N₂O₁₀, 588.3622, found 588.3626; calculated for C₂₆H₄₅N₂O₁₀, 545.3074, found 545.3077; calculated for C₂₅H₄₁N₂O₁₀, 529.2761, found 529.2758; calculated for C₁₁H₂₂NO₄, 232.1549, found 232.1542. Elemental analysis: Calculated for C₂₉H₅₂N₂O₁₀: C, 59.16; H, 8.90; N, 4.76; found C, 59.24; H, 8.90; N, 4.88.

### Example 3: Transformation of the scission-oxidation product of simple hydroxyproline derivatives into dipeptides.

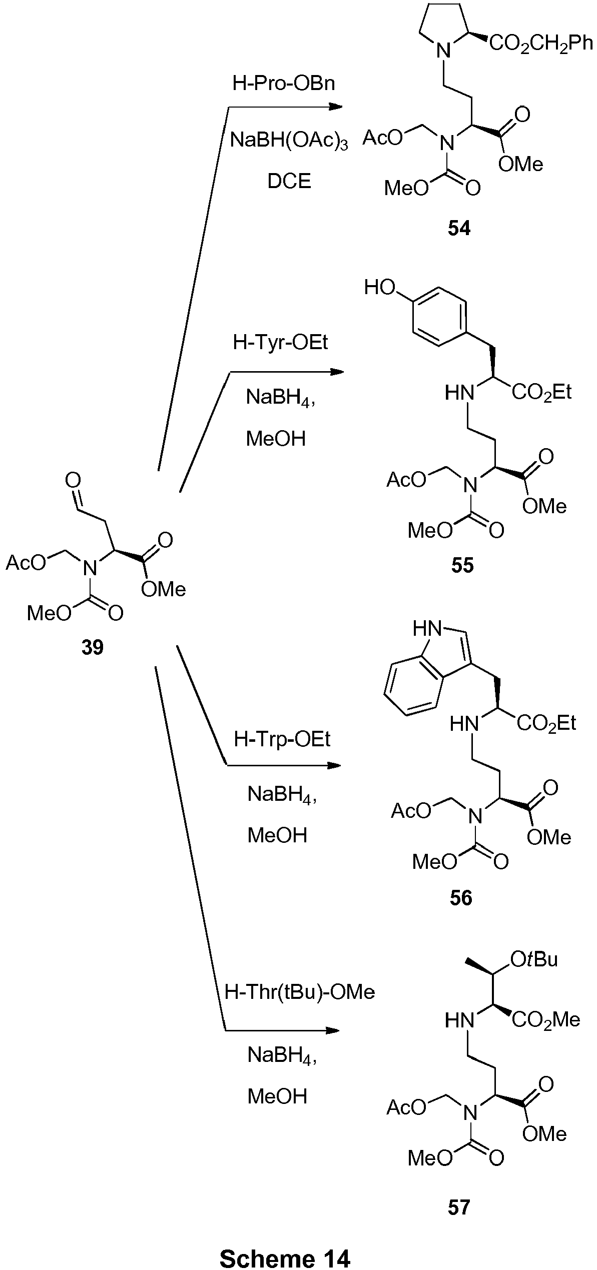

When the reductive amination process was carried out with α-amino esters (Scheme 14), such as H-Pro-OBn, H-Tyr-OEt, H-Trp-OEt, and H-Thr(tBu)OMe, the dipeptides (54)-(57) were obtained in high yields, and no epimerization was detected. The formation of these derivatives is relevant, since the 4-oxohomoalanine derivatives could be used as scaffolds for the attachment of peptide chains both at the terminal and the internal positions.

### Experimental Procedure for example 3: Reductive Amination Process of the Scission Products with α-amino esters, to give dipeptides.

*N-(N-Acetoxymethyl-N-methoxycarbonyl-1-methoxy-4-L-homoalanyl)-L-proline benzyl ester* (54). To a solution of the aldehyde (39) (53 mg, 0.20 mmol) in dry dichloroethane was added H-Pro-OBn·HCl (64 mg, 0.26 mmol) and triethylamine (38 µL, 0.27 mmol) and the mixture was stirred for 10 min. at 26 °C. Then sodium (triacetoxy)borohydride (69 mg, 0.32 mmol) was added and the stirring continued for 4.5 h. After usual work-up and concentration, the residue was purified by rotatory chromatography (hexane/EtOAc, 60:40), yielding the product (54) (73 mg, 80%), as a yellowish oil: [α]_{D} = -76.6 (c 0.34, CHCl₃); IR (CHCl₃) vₘₐₓ. 1740, 1721, 1447 cm⁻¹; ¹H NMR (500 MHz, CDCl₃, 26 °C) 4:1 rotamer mixture *δ*_{H} 1.82 (1H, m, 4'-Hₐ), 1.90-2.02 (2H, m, 4'-H_{b} + 3'-Hₐ), 2.02/2.05 (3H, s/s, Ac), 2.06-2.16 (2H, m, 3'-H_{b} + 3-Hₐ), 2.25 (1 H, m, 3-H_{b}), 2.34 (1 H, m, 5'-Hₐ), 2.46 (1 H, m, 4-Hₐ), 2.79 (1 H, m, 4-H_{b}), 3.15 (1 H, m, 5'-H_{b}), 3.24 (1 H, m, 2'-H), 3.67 (3H, s, OMe), 3.71/3.74 (3H, s/s, OMe), 4.55 (1 H, dd, *J* = 4.6, 10.0 Hz, 2-H), 5.11 (1H, d, *J* = 10.4 Hz, OCHₐPh), 5.14 (1H, d, *J* = 12.4 Hz, OCH_{b}Ph), 5.33-5.50 (2H, m, OCH₂N), 7.26-7.40 (5H, m, Ar); ¹³C NMR (125.7 MHz, CDCl₃, 26 °C) *δ*_{C} 20.9 (CH₃), 23.1 (CH₂), 28.2 (CH₂), 29.2 (CH₂), 50.5 (CH₂), 52.3 (CH₃), 52.8 (CH₂), 53.3/53.5 (CH₃), 58.2/58.5 (CH), 65.6 (CH), 66.2 (CH₂), 72.0/72.4 (CH₂), 128.2 (2 x CH + CH), 128.5 (2 x CH), 135.8 (C), 155.7 (C), 170.7 (C), 171.6 (C), 173.5 (C); MS (EI) m/z (relative intensity) 419 (M⁺ - OMe, <1), 391 (M⁺ - CO₂Me, 3), 315 (M⁺ - CO₂CH₂Ph, 100). HRMS calcd for C₂₁H₂₇N₂O₇, 419.1818, found 419.1804; calculated for C₂₀H₂₇N₂O₆, 391.1869, found 391.1872; calculated for C₁₄H₂₃N₂O₆, 315.1556, found 315.1559. Elemental analysis: Calculated for C₂₂H₃₀N₂O₈: C, 58.66; H, 6.71; N, 6.22; found C, 58.64; H, 6.89; N, 6.39.

*N-(N-Acetoxymethyl-N-methoxycarbonyl-1-methoxy-4-L-homoalanyl)-L-tyrosine ethyl ester* (55). To a solution of the aldehyde (39) (53 mg, 0.20 mmol) in dry methanol (3 mL) was added H-Tyr-OEt·HCl (70 mg, 0.28 mmol) and triethylamine (38 µL, 0.27 mmol) and the mixture was stirred for 1 h at 26 °C. Then sodium borohydride (10 mg, 0.26 mmol) was added and the stirring continued for 7 h. The mixture was poured into water and extracted with EtOAc. The organic layer was dried, concentrated and purified by rotatory chromatography (hexane/EtOAc, 70:30), affording the product (55) (72 mg, 78%), as a yellowish oil: [α]_{D} = - 27.4 (c 0.07, CHCl₃); IR (CHCl₃) vₘₐₓ. 3388, 1747, 1710, 1477 cm⁻¹; ¹H NMR (500 MHz, CDCl₃, 26 °C) 2:1 rotamer mixture *δ*_{H} 1.18 (3H, dd, *J* = 7.1, 7.2 Hz, OEt), 1.94 (1 H, brb, 3-Hₐ), 2.01 (3H, s, Ac), 2.13 (1 H, m, 3-H_{b}), 2.56 (1 H, m, 4-Hₐ), 2.67 (1 H, m, 4-H_{b}), 2.82 (1H, dd, *J* = 7.0, 13.7 Hz, 3'-Hₐ), 2.89 (1 H, dd, *J* = 6.5, 13.7 Hz, 3'-H_{b}), 3.44 (1 H, dd, *J* = 6.7, 7.5 Hz, 2'-H), 3.65 (3H, s, OMe), 3.67/3.73 (3H, brs, OMe), 4.10 (2H, ddd, *J* = 7.1, 7.1, 7.1 Hz, OEt), 4.43/4.50 (1 H, m/m, 2-H), 5.26/5.31 (1 H, [d, *J* = 11.4 Hz / d, *J =* 11.0 Hz], OCHₐN), 5.32/5.38 (1 H, [d, *J* = 10.8 Hz/ d, *J* = 11.1 Hz], OCH_{b}N), 6.69 (1 H, d, *J* = 8.5 Hz, Ar), 6.98 (1 H, d, *J* = 8.5 Hz, Ar); ¹³C NMR (125.7 MHz, CDCl₃, 26 °C) *δ*_{C} 14.1 (CH₃), 20.8 (CH₃), 29.3/30.0 (CH₂), 38.5 (CH₂), 44.1 (CH₂), 52.4 (CH₃), 53.4/53.5 (CH₃), 57.9 (CH), 60.9 (CH₂), 62.5 (CH), 71.6/72.4 (CH₂), 115.3 (2 x CH), 128.2 (C), 130.3 (2 x CH), 155.1 (C), 156.0/156.4 (C), 170.8/171.0 (C), 171.6 (C), 174.2 (C); MS (EI) m/z (relative intensity) 455 (M⁺ + H, 1), 395 (M⁺-CO₂Me, 9), 321 (M⁺ - CH₂OAc - CO₂Me - H, 18), 287 (M⁺ - CH₂Ph-OH-CO₂Me - H, 100). HRMS calcd for C₂₁H₃₁N₂O₉, 455.2030, found 455.2017; calculated for C₁₉H₂₇N₂O₇, 395.1818, found 391.1831; calculated for C₁₆H₂₁N₂O₅, 321.1450, found 321.1436; calculated for C₁₂H₁₉N₂O₆, 287.1243, found 287.1240. Elemental analysis: Calculated for C₂₁H₃₀N₂O₉: C, 55.50; H, 6.65; N, 6.16; found C, 55.61; H, 6.82; N, 5.90.

*N-(N-Acetoxymethyl-N-methoxycarbonyl-1-methoxy-4-L-homoalanyl)-L-tryptophan ethyl ester* (56). To a solution of the aldehyde (39) (53 mg, 0.20 mmol) in dry methanol (3 mL) was added H-Trp-OEt·HCl (77 mg, 0.29 mmol) and triethylamine (38 µL, 0.27 mmol) and the mixture was stirred for 60 min. at 26 °C. Then sodium borohydride (10 mg, 0.26 mmol) was added and the stirring continued for 8 h. The mixture was poured into water and extracted with EtOAc. The organic layer was dried, filtered and concentrated as usual. The residue was purified by rotatory chromatography (hexane/EtOAc, 70:30), affording the product (56) (77 mg, 80%), as a yellowish slurry. [α]_{D} = -21.35 (c 0.17, CHCl₃); IR (CHCl₃) vₘₐₓ. 1737, 1721, 1224, 1203 cm⁻¹; ¹H NMR (500 MHz, CDCl₃, 26 °C) 2:1 rotamer mixture *δ*_{H} 1.26 (3H, dd, *J* = 7.1, 7.2 Hz, OEt), 2.06 (1 H, m, 3-Hₐ), 2.07 (3H, s, Ac), 2.29 (1 H, m, 3-H_{b}), 2.72 (1 H, m, 4-Hₐ), 2.82 (1 H, m, 4-H_{b}), 3.23 (1 H, dd, *J* = 6.9, 14.5 Hz, CHₐAr), 3.35 (1 H, m, CH_{b}Ar), 3.71 (3H, brs, OMe), 3.76/3.80 (3H, s/s, OMe), 4.21 (2H, ddd, *J* = 7.1, 7.2, 7.2 Hz, OCH₂Me), 4.63 (1 H, m, CHN), 5.37/5.39 (1 H, [d, *J* = 11.4 Hz/ brb], CHₐAr), 5.46/5.48 (1 H, [d, *J =* 11.0 Hz/ m], CH_{b}Ar), 7.08 (1 H, dd, *J* = 8.0, 8.0 Hz, Ar), 7.11 (1 H, brb, 2'-H), 7.14 (1 H, dd, *J* = 7.8, 8.0 Hz, Ar), 7.33 (1 H, d, *J* = 8.1 Hz, Ar), 7.57 (1 H, d, *J* = 7.9 Hz, Ar), 8.52 (1 H, brs, CHO); ¹³C NMR (125.7 MHz, CDCl₃, 26 °C) *δ*_{C} 14.0 (CH₃), 20.8 (CH₃), 29.0/29.2 (CH₂), 29.6/30.1 (CH₂), 44.2 (CH₂), 52.3 (CH₃), 53.4 (CH₃), 58.1 (CH), 60.9 (CH₂), 61.3 (CH₃), 71.6/72.5 (CH₂), 110.4/111.2 (CH), 118.6 (CH), 119.3 (CH), 121.9 (CH), 123.5 (CH), 127.4 (C), 136.2 (C), 155.9/156.3 (C), 170.7/170.9 (C), 171.5 (C), 174.0 (C); MS (EI) m/z (relative intensity) 417 (M⁺ - AcOH, 9), 344 (M⁺ - CO₂Me - CH₂OAc, 15), 287 (M⁺ - CH₂Ar - AcOH, 100). HRMS calcd for C₂₁H₂₇N₃O₆, 417.1900, found 417.1915; calculated for C₁₈H₂₂N₃O₄, 344.1610, found 344.1614; calculated for C₁₂H₁₉N₂O₆, 287.1243, found 287.1252. Elemental analysis: Calculated for C₂₃H₃₁N₃O₈: C, 57.85; H, 6.54; N, 8.80; found C, 57.70; H, 6.870; N, 8.68.

*N-(N-Acetoxymethyl-N-methoxycarbonyl-1-methoxy-4-L-homoalanyl)-O-tert-butyl)-L-threonine methyl ester* (57). To a solution of the aldehyde (39) (53 mg, 0.20 mmol) in dry methanol (3 mL) was added H-Thr(*^{t}*Bu)-OMe·HCl (64 mg, 0.28 mmol) and triethylamine (38 µL, 0.27 mmol) and the mixture was stirred for 60 min. at 26 °C. Then sodium borohydride (10 mg, 0.26 mmol) was added and the stirring continued for 5 h. The mixture was poured into water and extracted with EtOAc. The organic layer was dried, filtered and concentrated as usual. The residue was purified by rotatory chromatography (hexane/EtOAc, 85:15), affording the product (57) (73 mg, 83%), as a yellowish slurry: [α]_{D} = -41.76 (c 0.33, CHCl₃); IR (CHCl₃) v_{max.} 1740, 1476, 1442 cm⁻¹; ¹H NMR (500 MHz, CDCl₃, 70 °C) *δ*_{H} 1.18 (9H, s, *t*Bu), 1.34 (3H, d, *J* = 6.3 Hz, 3'-Me), 2.06 (3H, s, Ac), 2.07 (1H, brb, 3-Hₐ), 2.50 (1H, brb, 3-H_{b}), 2.65 (1H, brb, 4-Hₐ), 2.96 (1H, brb, 4-H_{b}), 3.42 (1 H, brb, 2'-H), 3.72 (3H, s, OMe), 3.76 (6H, s, 2 x OMe), 4.20 (1 H, m, 3'-H), 4.68 (1 H, br dd, *J* = 6.9, 7.1 Hz, 2-H), 5.40 (1 H, d, *J* = 11.2 Hz, OCHₐN), 5.48 (1 H, d, *J* = 11.2 Hz, OCH_{b}N); ¹³C NMR (125.7 MHz, CDCl₃, 26 °C) rotamer mixture *δ*_{C} 20.6/20.9 (CH₃), 28.6 (3 x CH₃), 29.6/30.4 (CH₂), 44.5 (CH₂), 51.8 (CH₃), 52.4 (CH₃), 53.4/53.5 (CH₃), 58.0 (CH), 66.6 (CH), 68.2 (CH), 71.6/72.4 (CH₂), 74.0 (C), 156.0 (C), 170.7 (C), 171.0 (C), 171.8 (C); MS (EI) m/z (relative intensity) 375 (M⁺ - CO₂Me, <1), 315 (M⁺ - AcOH - CO₂Me, 5), 301 (M⁺ - CH₂OAc - CO₂Me - H, 9), 273 (M⁺ - AcOCH₂NCO₂Me - Me, 100), 57 (*t*Bu, 91). HRMS calcd for C₁₇H₃₁N₂O₇ 375.2131, found 375.2138; calculated for C₁₅H₂₇N₂O₅ 315.1920, found 315.1929; calculated for C₁₄H₂₅N₂O₅ 301.1763, found 301.1778; calculated for C₁₃H₂₃NO₅ 273.1576, found 273.1588; calculated for C₄H₉, 57.0704, found 57.0702. Elemental analysis: Calculated for C₁₉H₃₄N₂O₉: C, 52.52; H, 7.89; N, 6.45; found C, 52.33; H, 7.64; N, 6.44.

### Example 4: Extension of the alkyl chain of the scission products derived from 4-hydroxy-L-hydroxyproline.

The lateral chain of the scission products can be readily transformed into other carbon chains using a Wittig or a Horner-Wadsworth-Emmons reaction. Scheme 15 shows the conversion of aldehyde (39) into a valuable 4,5-dehydro-L-homoglutamate (58), which was reduced to the high-profit L-homoglutamate derivative (59). The procedure is applicable to the preparation of other alkyl or olefinic chains.

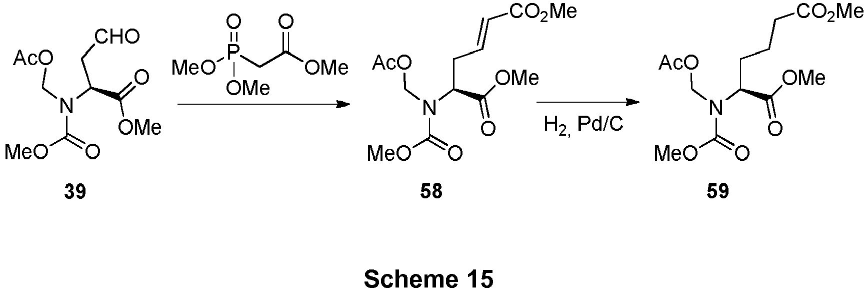

### Experimental procedure for example 4: Horner-Wadsworth-Emmons reaction of the scission products, followed by hydrogenation, to give homoglutamate derivatives.

*Dimethyl N-Acetoxymethyl-N-(methoxycarbonyl)-4,5-dehydro-L-homoglutamate* (58). A suspension of sodium hydride (60 % dispersion in mineral oil, 6 mg, 0.15 mmol) in dry THF (1.5 mL) was cooled to -10 °C, treated dropwise with a solution of methyl 2-(dimethoxyphosphoryl)acetate (27 mg, 0.15 mmol) in dry THF (1 mL), and stirred for 45 minutes. Then a solution of aldehyde (39) (27 mg, 0.10 mmol) in dry THF (1 mL) was slowly added at -10 °C, while stirring for 35 min. The reaction mixture was poured into water and extracted with diethyl ether. The organic layer was dried, filtered and concentrated as usual, and the residue was purified by rotatory chromatography (hexane/EtOAc, 80:20), yielding product (58) (26 mg, 79%) as a yellowish slurry. [α]_{D} =-37.32 (*c* 0.41, CHCl₃); IR (CHCl₃) vₘₐₓ. 3026, 1720, 1440 cm⁻¹; ¹H NMR (500 MHz, CDCl₃, 70 °C) *δ*_{H} 2.03 (3H, s, Ac), 2.82 (1 H, m, 3-Hₐ), 2.92 (1 H, m, 3-H_{b}), 3.70 (3H, s, OMe), 3.71 (3H, s, OMe), 3.76 (3H, s, OMe), 4.47 (1 H, dd, *J =* 5.6, 9.6 Hz, 2-H), 5.34 (1H, d, *J* = 11.3 Hz, OCHₐN), 5.40 (1H, d, *J* = 11.3 Hz, OCH_{b}N), 5.88 (1 H, d, *J* = 15.7 Hz, 5-H), 6.85 (1 H, ddd, *J* = 7.1, 7.5, 15.7 Hz, 4-H); ¹³C NMR (125.7 MHz, CDCl₃, 26 °C) *δ*_{C} 20.6 (CH₃), 32.5 (CH₂), 51.3 (CH₃), 52.4 (CH₃), 53.4 (CH₃), 59.7 (CH), 71.7 (CH₂), 124.0 (CH), 143.7 (CH), 155.8 (C), 166.3 (C), 170.3 (C), 170.5 (C); MS (EI) m/z (relative intensity) 317 (M⁺, 2), 258 (M⁺ - CO₂Me, 11), 218 (M⁺ - CH₂CH=CH-CO₂Me, 64), 198 (M⁺ - (2 x CO₂Me) - H, 100); HRMS calcd for C₁₃H₁₉NO₈, 317.1111, found 317.1112; calculated for C₁₁H₁₆NO₆, 258.0978, found 258.0975; calculated for C₈H₁₂NO₆, 218.0665, found 218.0665; calculated for C₉H₁₂NO₄, 198.0766, found 198.0767. Elemental analysis: Calculated for C₁₃H₁₉NO₈: C, 49.21; H, 6.04; N, 4.41; found C, 49.54; H, 6.32; N, 4.45.

*Dimethyl N-acetoxymethyl-N-(methoxycarbonyl)-L-homoglutamate* (59). A solution of the dehydroamino ester (58) (14 mg, 0.044 mmol) in MeOH (5 mL) was treated with Pd (10% on C, 5 mg). The mixture was stirred under hydrogen atmosphere (1 atm) for 18 h, then was filtered on celite and eluted with EtOAc. The organic layer was concentrated under vacuum, yielding the reduction product (59) (13 mg, 93%) as a yellowish oil: [α]_{D} = -28.22 (c 0.39, CHCl₃); IR (CHCl₃) vₘₐₓ. 1737, 1442 cm⁻¹; ¹H NMR (500 MHz, CDCl₃, 26 °C) *δ*_{H} 1.66-1.80 (2H, m, 4-H₂), 1.87 (1 H, m, 3-Hₐ), 2.05 (1 H, m, 3-H_{b}), 2.04 (3H, s, Ac), 2.28-2.40 (2H, m, 5-H₂), 3.66 (3H, s, OMe), 3.70 (3H, s, OMe), 3.76 (3H, s, OMe), 4.50 (1 H, m, 2-H), 5.35 (1 H, d, *J* = 11.3 Hz, OCHₐN), 5.49 (1 H, d, *J* = 11.3 Hz, OCH_{b}N); ¹³C NMR (125.7 MHz, CDCl₃, 26 °C) *δ*_{C} 20.7 (CH₃), 21.9 (CH₂), 29.4 (CH₂), 33.6 (CH₂), 51.4 (CH₃), 52.2 (CH₃), 53.3 (CH₃), 59.7 (CH), 71.2 (CH₂), 156.3 (C), 170.4 (C), 171.3 (C), 173.2 (C); MS (EI) m/z (relative intensity) 288 (M⁺ - OMe, 2), 260 (M⁺ - CO₂Me, 11), 259 (M⁺ - CO₂Me - H, 14), 200 (M⁺ - CO₂Me - AcOH, 100); HRMS calcd for C₁₂H₁₈NO₇, 288.1083, found 288.1069; calculated for C₁₁H₁₈NO₆, 260.1134, found 260.1134; calculated for C₁₁H₁₇NO₆, 259.1056, found 259.1047; calculated for C₉H₁₄NO₄, 200.0923, found 200.0927. Elemental analysis: Calculated for C₁₃H₂₁NO₈: C, 48.90; H, 6.63; N, 4.39; found C, 48.92; H, 6.42; N, 4.36.

### Example 5: Formation of N-methylated amino acids by reduction of the N,O-acetal group of the scission products derived from 4-hydroxy-L-proline.

The generation of *N*-substituted amino acids is important from a commercial standpoint, since there are relatively few commercially available *N*-alkyl amino acids and they reach a high commercial value. The acetoxymethyl group can be transformed into other groups by addition of nucleophiles or reduction. Because of the importance of *N*-methyl derivatives, a model compound (40) (Scheme 16) was reduced with triethylsilane. The reduction of the acetal was accompanied by reduction of the aldehyde and lactonization, affording the *N*-methyl homoserine lactone (61).

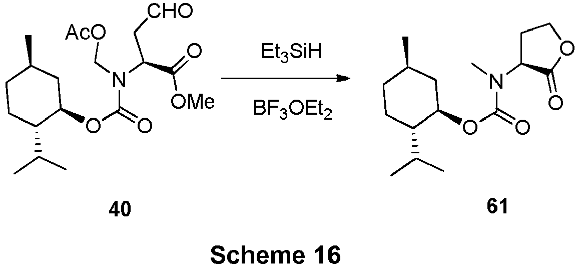

The reduction also took place in good yields with the dehydroglutamate derivative (58) (Scheme 17), yielding exclusively the *N*-methyl derivative (62).

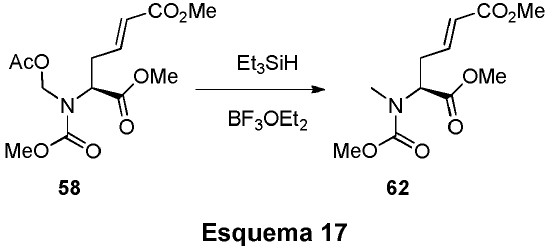

### Experimental procedure for example 5: Procedure for the reduction of the acetoxymethyl group.

*N-[(1R,2S,5R)-menthyloxycarbonyl]-N-methyl-L-homoserine lactone* (61): To a solution of the aldehyde (40) (39 mg, 0.10 mmol) in dry dichloromethane (2 mL) was added BF₃·OEt₂ (25 µL, 0.2 mmol) and Et₃SiH (40 µL, 0.25 mmol), and the mixture was stirred at 26 °C for 16 h. Then it was poured into a saturated aqueous NaHCO₃ solution and extracted with dichloromethane. The organic layer was dried, filtered and concentrated as usual, and the residue was purified by rotatory chromatography (hexane/EtOAc, 80:20), affording the *N*-methyl homoserine lactone (61) (21 mg, 70%) as a yellowish oil: [α]_{D} = -60.3 (c 0.34, CHCl₃); ¹H NMR (500 MHz, CDCl₃, 70 °C) *δ*_{H} 0.81 (3H, d, *J* = 7.0 Hz, Me), 0.87 (1 H, m, 4'-Hₐ), 0.91 (3H, d, *J* = 6.7 Hz, Me), 0.92 (3H, d, *J* = 7.0 Hz, Me), 0.98 (1H, ddd, *J* = 11.4, 11.7, 12 Hz, 6'-Hₐ), 1.08 (1H, dddd, *J* = 3.2, 12.6, 12.9, 13.0 Hz, 3'-Ha), 1.39 (1 H, br dd, *J* = 11, 12 Hz, 2'-H), 1.50 (1 H, m, 5'-H), 1.66-1.73 (2H, m, 3'-H_{b} + 4'-H_{b}), 1.92 (1 H, m, 2"-H), 2.09 (1 H, m, 6'-Hₐ), 2.38 (1 H, m, 4-Hₐ), 2.45 (1 H, m, 4-H_{b}), 2.92 (3H, s, NMe), 4.23 (1 H, ddd, *J* = 7.0, 9.5, 9.8 Hz, OCHₐ), 4.43 (1 H, ddd, *J* = 2.6, 9.2, 9.2 Hz, OCH_{b}), 4.63 (1 H, ddd, *J* = 4.4, 10.7, 11.1 Hz, 1'-H), 4.71 (1 H, br dd, *J* = 9.2, 9.8 Hz, CHN); ¹³C NMR (125.7 MHz, CDCl₃, 70 °C) *δ*_{C} 16.6 (CH₃), 20.8 (CH₃), 21.9 (CH₃), 24.0 (CH₂), 26.0 (CH₂), 26.6 (CH), 31.0 (CH), 31.5 (CH), 32.4 (CH₃), 34.5 (CH₂), 41.6 (CH₂), 47.7 (CH), 56.4 (CH), 65.1 (CH₂), 76.6 (CH), 156.1 (C), 173.4 (C); MS (EI) m/z (relative intensity) 297 (M⁺, <1), 239 (M⁺ - CO₂CH₂, 1), 83 ([cyclohexenyll + H]⁺, 100). HRMS calcd for C₁₆H₂₇NO₄, 297.1940, found 297.1946; calculated for C₁₄H₂₅NO₂ 239.1885, found 239.1889; calculated for C₆H₁₁, 83.0861, found 83.0858. Elemental analysis: Calculated for C₁₆H₂₇NO₄: C, 64.62; H, 9.15; N, 4.71; found C, 64.39; H, 9.14; N, 4.67.

*Dimethyl N-Methyl-N-(methoxycarbonyl)-4,5-dehydro-L-homoglutamate* (62). To a solution of the dehydroamino acid (58) (32 mg, 0.10 mmol) in dry CH₂Cl₂ (2 mL) was added BF₃·OEt₂ (25 µL, 0.2 mmol) and Et₃SiH (40 µL, 0.25 mmol), and the mixture was stirred at 26 °C for 9 h. Then it was poured into saturated aqueous NaHCO₃ and extracted with dichloromethane. The organic layer was dried, filtered and concentrated as usual, and the residue was purified by rotatory chromatography (hexane/EtOAc 90:10), affording the *N*-methyl derivative (62) (19 mg, 73%) as a yellowish oil; [α]_{D} = -23.73 (c 0.45, CHCl₃); ¹H NMR (500 MHz, CDCl₃, 70 °C) *δ*_{H} 2.67 (1 H, m, 3-Hₐ), 2.86 (1 H, m, 3-H_{b}), 2.85 (3H, s, NMe), 3.72 (6H, s, 2 x OMe), 3.73 (3H, s, OMe), 4.78 (1 H, m, 2-H), 5.91 (1 H, d, *J* = 17.1 Hz, 5-H), 6.87 (1 H, ddd, *J* = 7.6, 7.9, 14.5 Hz, 4-H); ¹³C NMR (125.7 MHz, CDCl₃, 70 °C) *δ*_{C} 31.4 (CH₂), 31.9 (CH₃), 51.3 (CH₃), 52.2 (CH₃), 52.9 (CH₃), 58.4 (CH), 124.0 (CH), 143.4 (CH), 166.3 (C), 170.7 (C); MS (EI) m/z (relative intensity) 259 (M⁺, <1), 200 (M⁺ - CO₂Me, 41), 160 (MeO₂C-N(Me)=CH-CO₂Me, 100); HRMS calcd for C₁₁H₁₇NO₆, 259.1056, found 259.1050; calculated for C₉H₁₄NO₄, 200.0923, found 200.0928; calculated for C₆H₁₀NO₄, 160.0610, found 160.0610. Elemental analysis: Calculated for C₁₁H₁₇NO₆: C, 50.96; H, 6.61; N, 5.40; found C, 50.67; H, 6.48; N, 5.19.

### Example 6: Application of the oxidative radical scission of hydroxyproline derivatives to the selective modification of dipeptides.

The oxidative radical scission process was then studied with small size peptides containing the hydroxyproline unit, such as the dipeptide (64) shown in Scheme 18. With peptide substrates, it was feared that side-reactions such as hydrogen abstraction might take place, thus decreasing the yields of the scission products. However, the scission of substrate (64) proceeded quickly and cleanly, and in order to facilitate handling of the scission product, the latter was immediately reduced to give the homoserine lactone (48).

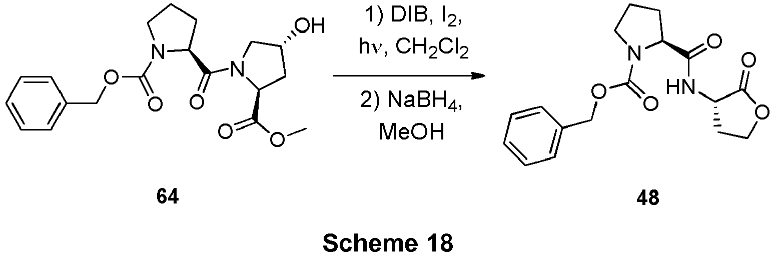

In another example, the dipeptide (65) (Scheme 19) was treated under the oxidative radical scission conditions, affording the 4-oxohomoalanine derivative (41). This product underwent a Horner-Wadsworth-Emmons reaction, to give the 3,4-dehydrohomoglutamate (60) in good yields and optical purity. Dehydro analogues of amino acids reach high commercial prices. Interestingly, the reaction conditions did not affect to the protecting groups and did not cause epimerization of the products.

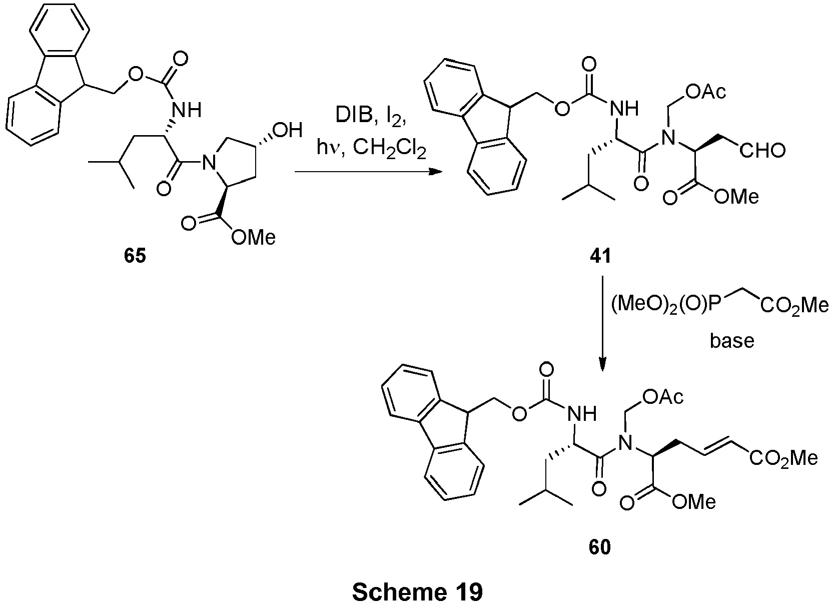

### Experimental procedure for example 6: Scission-oxidation of Dipeptide (55), followed by Reduction-Lactonization

*N-(N-Benzyloxycarbonyl-L-prolyl)-4R-hydroxy-L-proline methyl ester* (64). A solution of Cbz-Pro-OH (600 mg, 2.41 mmol) and H-Hyp-OMe·HCl (479 mg, 2.65 mmol) in CH₂Cl₂ (30 mL) at 0°C was treated with DIPEA (0.82 mL, 4.71 mmol), HOBt·H₂O (404 mg, 2.64 mmol) and EDAC (506 mg, 2.64 mmol). After stirring at 0°C for 1 h, the mixture was allowed to reach room temperature and then was stirred for other 20 h. Then it was poured into saturated aqueous NaHCO₃ and the organic layer was washed with 10% aqueous HCl, then dried and concentrated as usual. The residue was purified by column chromatography on silica gel (hexane/EtOAc, 40:60), yielding the dipeptide Cbz-Pro-Hyp-OMe (64) (753 mg, 83%), as a white foam: [α]_{D} = -98.9 (c 0.18, CHCl₃); IR (CHCl₃) vₘₐₓ. 3440, 1744, 1684, 1658, 1449, 1436 cm⁻¹; ¹H NMR (500 MHz, CDCl₃, 26 °C) 3:1 rotamer mixture *δ*_{H} 1.88 (1 H, m, 4'-Hₐ), 1.96 (1 H, ddd, *J* = 4.6, 8.5, 13.3 Hz, 3-Hₐ), 1.98-2.20 (3H, m, 3'-H₂ + 4'-H_{b}), 2.34 (1 H, m, 3-H_{b}), 3.50 (1 H, m, 5'-Hₐ), 3.54-3.68 (2H, m, 5'-H_{b} + 5-Hₐ), 3.70/3.71 (3H, s, OMe), 4.01 (1 H, d, J = 10.9 Hz, 5-Hb), 4.43-4.53 (2H, m, 2'-H + 4-H), 4.70 (1 H, dd, J = 8.3, 8.3 Hz, 2-H), 5.03/5.04 (1 H, [d, *J =* 12.5 Hz/ d, *J* = 12.4 Hz], OCHₐPh), 5.10/5.14 (1 H, [d, *J* = 12.3 Hz/ d, *J* = 12.5 Hz], OCH_{b}Ph), 7.30-7.34 (5H, m, Ar); ¹³C NMR (125.7 MHz, CDCl₃, 26 °C) rotamer mixture *δ*_{C} 23.5/24.3 (CH₂), 29.3/30.1 (CH₂), 37.0/37.6 (CH₂), 46.9/47.2 (CH₂), 52.3 (CH₃), 54.4/54.9 (CH₂), 57.6 (CH), 57.9 (CH), 67.0/67.2 (CH₂), 70.3/70.5 (CH), 127.7 (2 x CH), 128.0 (CH), 128.5 (2 x CH), 136.5/136.9 (C), 154.2/155.2 (C), 171.3/171.6 (C), 172.6/172.9 (C); MS (EI) m/z (relative intensity) 376 (M⁺, 1), 204 (M⁺ - CO-Hyp-OMe, 22), 91 ([OCH₂Ph]⁺, 100). HRMS calcd for C₁₉H₂₄N₂O₆, 376.1634, found 376.1628; calculated for C₁₂H₁₄NO₂, 204.1025, found 204.1018; calculated for C₇H₇, 91.0548, found 91.0552. Elemental analysis: Calculated for C₁₉H₂₄N₂O₆: C, 60.63; H, 6.43; N, 7.44; found C, 60.31; H, 6.53; N, 7.28.

*N-(N-Benzyloxycarbonyl-L-prolyl)-L-homoserine lactone* (48). A solution of the dipeptide Cbz-Pro-Hyp-OMe (64) (300 mg, 0.80 mmol) in dry DCM (10 mL) was treated with (diacetoxyiodo)benzene (508 mg, 1.58 mmol) and iodine (100 mg, 0.39 mmol). The mixture was stirred at room temperature for 4 h under irradiation with visible light (80W tungsten-filament lamp), then was poured into 10% aqueous Na₂S₂O₃ and extracted with dichloromethane. The organic layer was dried, filtered and concentrated as usual, and the residue was purified by column chromatography (hexane/EtOAc, 60:40), yielding the corresponding aldehyde (215 mg, 62%), as a yellowish gum. Part of this intermediate aldehyde (44 mg, 0.10 mmol) was dissolved in dry methanol (2 mL), and treated with sodium borohydride (5 mg, 0.13 mmol). The reaction mixture was heated to 45°C and stirred for 20 h, and then was allowed to cool to room temperature, poured into water and extracted with EtOAc. The organic layer was dried, filtered and concentrated as usual, and the residue was purified by column chromatography (hexane/EtOAc, 50:50), affording the lactone (48) (27 mg, 80%), as a yellowish foam: [α]_{D} = -68.75 (c 0.2, CHCl₃); IR (CHCl₃) vₘₐₓ. 3419, 1783, 1729, 1688 cm⁻¹; ¹H NMR (500 MHz, CDCl₃, 70 °C) *δ*_{H} 1.89 (1H, m, 4'-Hₐ), 1.94-2.14 (3H, m, 4'-H_{b} + 3'-H_{b} + 3-H_{b}), 2.28 (1 H, m, 3'-Hₐ), 2.65 (1 H, m, 3-Hₐ), 3.48-3.58 (2H, m, 5'-H₂), 4.20 (1 H, ddd, *J* = 6.3, 9.3, 10.6 Hz, 4-Hₐ), 4.32-4.44 (3H, m, 4-H_{b} + 2'-H + 2-H), 5.13 (1 H, d, *J* = 12.4 Hz, OCHₐPh), 5.18 (1 H, d, *J* = 12.4 Hz, OCH_{b}Ph), 7.27-7.40 (5H, m, Ar); ¹³C NMR (125.7 MHz, CDCl₃, 70 °C) *δ*_{C} 24.3 (CH₂), 29.8 (2 x CH₂), 47.3 (CH₂), 49.2 (CH), 60.6 (CH), 65.7 (CH₂), 67.5 (CH₂), 128.0 (2 x CH), 128.1 (CH), 128.5 (2 x CH), 136.5 (C), 172.5 (C), 174.5 (C); MS (EI) m/z (relative intensity) 332 (M⁺, <1), 204 ([Cbz-pyrrolidine - H]⁺, 22), 91 ([CH₂Ph]⁺, 100). HRMS calcd for C₁₇H₂₀N₂O₅, 332.1372, found 332.1375; calculated for C₁₂H₁₄NO₂, 204.1025, found 204.1018; calculated for C₇H₇, 91.0548, found 91.0545.

### Experimental procedure for example 6: Scission-oxidation of dipeptide (65) followed by a Horner-Wadsworth-Emmons reaction

*N-(N-Fluorenylmethyloxycarbonyl-L-leucyl)-4R-hydroxy-L-proline methyl ester* (65). A solution of Fmoc-Leu-OH (700 mg, 1.98 mmol) and H-Hyp-OMe·HCl (400 mg, 2.20 mmol) in dichloromethane (30 mL) at 0°C was treated with DIPEA (0.67 mL, 3.85 mmol), HOBt (333 mg, 2.17 mmol) and EDAC (417 mg, 2.17 mmol). The reaction mixture was stirred at 0°C for 1 h and then was allowed to reach room temperature and stirred for other 20 h. The mixture was poured into saturated aqueous NaHCO₃, and the organic layer was separated, washed with 10% aqueous HCl, and finally dried and concentrated as usual. The residue was purified by column chromatography (hexane/EtOAc, 60:40), yielding the dipeptide (65) (771 mg, 81%), as a white foam: [α]_{D} = -58.13 (c 0.16, CHCl₃); IR (CHCl₃) vₘₐₓ. 3430, 1745, 1711, 1650, 1511 cm⁻¹; ¹H NMR (400 MHz, CDCl₃, 70 °C) *δ*_{H} 0.96 (3H, d, *J* = 6.7 Hz, Me), 0.98 (3H, d, *J* = 6.5 Hz, Me), 1.50-1.65 (2H, m, 3'-H₂), 1.75 (1 H, m, 4'-H), 2.01 (1 H, m, 3-Hₐ), 2.31 (1 H, br dd, *J* = 8.2, 13.4 Hz, 3-H_{b}), 2.89 (1 H, br b, OH), 3.60-3.75 (1 H, m, 5-Hₐ), 3.72 (3H, s, OMe), 3.91 (1 H, d, *J* = 11.1 Hz, 5-H_{b}), 4.17 (1 H, dd, *J* = 7.2, 7.5 Hz, CH_{Fmoc}), 4.25-4.40 (2H, m, CH₂O), 4.45-4.55 (2H, m, 2'-H/2-H), 4.66 (1 H, dd, *J* = 8.2, 8.4 Hz, 4-H), 5.59 (1 H, d, *J* = 8.5 Hz, NH), 7.29 (2H, dd, *J* = 7.4, 7.5 Hz, Ar), 7.38 (2H, dd, *J* = 7.5, 7.5 Hz, Ar), 7.55-7.57 (2H, m, Ar), 7.74 (2H, d, *J* = 7.5 Hz, Ar); ¹³C NMR (100.6 MHz, CDCl₃, 26 °C) *δ*_{C} 22.0 (CH₃), 23.1 (CH₃), 24.5 (CH), 37.5 (CH₂), 41.6 (CH₂), 47.1 (CH), 50.9 (CH), 52.2 (CH₃), 55.2 (CH₂), 57.6 (CH), 67.2 (CH₂), 70.3 (CH), 119.9 (2 x CH), 125.1 (2 x CH), 127.1 (2 x CH), 127.7 (2 x CH), 141.3 (2 x C), 143.7/143.8 (2 x C), 156.5 (C), 171.8 (C), 172.4 (C); MS (EI) m/z (relative intensity) 480 (M⁺, <1), 226 (M⁺ - fluorenylmethyl-OCO - HOMe, 17), 196 ([fluorenyl-CH₂OH]⁺, 16), 179 ([fluorenyl-CH₂]⁺, 100), 178 ([fluorenyl-CH₂ - H]⁺, 77); HRMS calcd for C₂₇H₃₂N₂O₆, 480.2260, found 480.2242; calculated for C₁₁H₁₈N₂O₃, 226.1317, found 226.1321; calculated for C₁₀H₁₄N₂O, 178.1106, found 178.1098. Elemental analysis: Calculated for C₂₇H₃₂N₂O₆: C, 67.48; H, 6.71; N, 5.83; found C, 67.78; H, 6.67; N, 5.55.

*N-Acetoxym eth yl-N-(N-fluoren ylm eth yloxycarbon yl-L-leucyl)-4-oxo-L-homoalanine methyl ester* (41). A solution of dipeptide (65) (460 mg, 0.96 mmol) in dry DCM (10 mL), was treated with (diacetoxyiodo)benzene (612 mg, 1.90 mmol) and iodine (121 mg, 0.48 mmol). The mixture was stirred at room temperature for 4 h under irradiation with visible light. After usual work-up and concentration, the residue was purified by column chromatography (hexane/EtOAc, 70:30), affording the aldehyde (41) (315 mg, 61%), as a yellowish gum: [α]_{D} =-35.74 (*c* 0.15, CHCl₃); IR (CHCl₃) vₘₐₓ. 3430, 1745, 1724, 1669, 1570 cm⁻¹; ¹H NMR (500 MHz, CDCl₃, 70 °C) *δ*_{H} 0.94 (3H, d, *J* = 6.7 Hz, Me), 1.00 (3H, d, *J* = 6.6 Hz, Me), 1.51 (2H, m, 3'-H₂), 1.68 (1 H, m, 4'-H), 2.09 (3H, s, Ac), 3.03 (1 H, dd, *J* = 7.5, 18.8 Hz, 3-Hₐ), 3.39 (1H, dd, *J* = 5.3, 18.6 Hz, 3-H_{b}), 3.66 (3H, s, OMe), 4.22 (1 H, dd, *J* = 7.1, 7.2 Hz, CH_{Fmoc}), 4.36 (1H, dd, *J* = 7.1, 10.2 Hz, OCHₐ-fluorenyl), 4.41 (1 H, dd, *J* = 7.2, 10.5 Hz, OCH_{b}-fluorenyl), 4.80-4.92 (2H, m, 2'-H + 2-H), 5.34 (1 H, d, *J =* 8.9 Hz, NH), 5.46 (1H, d, *J =* 12.2 Hz, NCHₐO), 5.64 (1 H, d, *J* = 12.3 Hz, NCH_{b}N), 7.31 (2H, dd, *J* = 7.5, 7.5 Hz, Ar), 7.40 (2H, dd, *J* = 7.3, 7.6 Hz, Ar), 7.59 (2H, d, *J* = 7.5 Hz, Ar), 7.76 (2H, d, *J* = 7.6 Hz, Ar), 9.75 (1 H, s, CHO); ¹³C NMR (125.7 MHz, CDCl₃, 26 °C) *δ*_{C} 20.6 (CH₃), 21.7 (CH₃), 23.3 (CH₃), 24.7 (CH), 42.7 (CH₂), 43.9 (CH₂), 47.2 (CH), 49.8 (CH), 52.7 (CH₃), 55.5 (CH), 67.0 (CH₂), 72.1 (CH₂), 120.0 (2 x CH), 125.1 (2 x CH), 127.1 (2 x CH), 127.7 (2 x CH), 141.3 (2 x C), 143.8 (2 x C), 155.9 (C), 169.7 (C), 170.4 (C), 174.7 (C), 198.4 (CH); MS (EI) m/z (relative intensity) 538 (M⁺, <1), 308 ([Fmoc-NHCHCH₂CHMe₂]+, 4), 180 ([fluorenyl-CH₂ + H]⁺, 7), 178 ([fluorenyl-CH₂ - H]⁺, 100); HRMS calcd for C₂₉H₃₄N₂O₈, 538.2315, found 538.2332; calculated for C₂₀H₂₂NO₂, 308.1651, found 308.1654; calculated for C₁₄H₁₁ 179.0861, found 179.0861; calculated for C₁₄H₁₀ 178.0783, found 178.0782. Elemental analysis: Calculated for C₂₉H₃₄N₂O₈: C, 64.67; H, 6.36; N, 5.20; found C, 64.51; H, 6.19; N, 5.54.

*Dimethyl N-Acetoxymethyl-N-(N-fluorenylmethyloxycarbonyl-L-leucyl)-N-acetoxymethyl-4,5-dehydro-L-homoglutamate* (60). A suspension of sodium hydride (60 % in mineral oil, 6 mg, 0.15 mmol) in dry THF (1.5 mL) was cooled to -10 °C and a solution of methyl 2-(dimethoxyphosphoryl)acetate (27 mg, 0.15 mmol) in dry THF (1 mL) was added dropwise. The mixture was stirred for 45 minutes, and then a solution of the aldehyde (41) (54 mg, 0.10 mmol) in dry THF (1 mL) was slowly added, while the temperature was mantained at -10 °C for 35 minutes. The reaction mixture was poured into water and extracted with diethyl ether. The organic layer was dried, filtered and evaporated as usual, and the residue was purified by rotatory chromatography (hexane/EtOAc, 80:20), affording the olefin (60) (49 mg, 82%) as a yellowish slurry: [α]_{D} = -22.30 (c 0.38, CHCl₃); IR (CHCl₃) v_{max.} 3431, 1745, 1721, 1674, 1511 cm⁻¹; ¹H NMR (500 MHz, CDCl₃, 26 °C) *δ*_{H} 0.94 (3H, d, *J* = 6.7 Hz, Me), 0.98 (3H, d, *J* = 6.6 Hz, Me), 1.42-1.56 (2H, m, 3'-H₂), 1.70 (1 H, m, 4'-H), 2.10 (3H, s, Ac), 2.92 (2H, dd, *J =* 7.3, 7.4 Hz, 3-H₂), 3.67 (3H, s, OMe), 3.68 (3H, s, OMe), 4.21 (1 H, dd, *J* = 7.2, 7.2 Hz, CH_{Fmoc}), 4.34 (1 H, dd, *J =* 7.0, 10.6 Hz, OCHₐ-fluorenyl), 4.40 (1 H, dd, *J* = 7.4, 10.6 Hz, OCH_{b}-fluorenyl), 4.55 (1 H, dd, *J* = 7.0, 8.3 Hz, 2-H), 4.83 (1 H, ddd, *J* = 4.2, 9.1, 9.4 Hz, 2'-H), 5.34 (1 H, d, *J* = 9.1 Hz, NH), 5.43 (1 H, d, *J =* 12.4 Hz, NCHₐO), 5.55 (1 H, d, *J* = 12.4 Hz, NCH_{b}O), 5.85 (1 H, d, *J* = 15.6 Hz, 5-H), 6.80 (1 H, ddd, *J* = 7.6, 8.1, 15.6 Hz, 4-H), 7.30 (2H, dd, *J* = 7.5, 7.5 Hz, Ar), 7.39 (2H, dd, *J* = 7.4, 7.5 Hz, Ar), 7.58 (1 H, d, *J* = 7.5 Hz, Ar), 7.75 (1 H, d, *J =* 7.6 Hz, Ar); ¹³C NMR (125.7 MHz, CDCl₃, 26 °C) *δ*_{C} 20.6 (CH₃), 21.6 (CH₃), 23.2 (CH₃), 24.7 (CH), 31.6 (CH₂), 42.9 (CH₂), 47.2 (CH), 49.9 (CH), 51.4 (CH₃), 52.6 (CH₃), 59.6 (CH), 67.1 (CH₂), 71.7 (CH₂), 120.0 (2 x CH), 124.0 (CH), 125.1 (2 x CH), 127.0 (2 x CH), 127.7 (2 x CH), 141.3 (2 x C), 143.8 (CH), 143.9 (2 x C), 156.5 (C), 166.3 (C), 169.9 (C), 170.5 (C), 174.9 (C); MS (EI) m/z (relative intensity) 594 (M⁺, <1), 339 (M⁺ - fluorenylmethyl -OCO - HOMe, <1), 308 ([Fmoc-NH-CHCH₂CHMe₂]+, 3), 279 ([Fmoc-NH-CHCO]+, 3), 200 ([CH₂=NH-CH(CO₂Me)CH₂CH=CHCO₂Me + H]⁺, 6), 179 ([fluorenyl-CH₂]⁺, 60), 178 ([fluorenyl-CH₂ - H]⁺, 100), 165 ([fluorenyl]⁺, 25); HRMS calcd for C₃₂H₃₈N₂O₉, 594.2577, found 594.2573; calculated for C₁₆H₂₃N₂O₆, 339.1556, found 339.1564; calculated for C₂₀H₂₂NO₂, 308.1651, found 308.1647; calculated for C₁₄H₁₁, 179.0861, found 179.0857; calculated for C₁₄H₁₀, 178.0783, found 178.0790; calculated for C₁₃H₉, 165.0704, found 165.0707. Elemental analysis: Calculated for C₃₂H₃₈N₂O₉: C, 64.63; H, 6.44; N, 4.71; found C, 64.75; H, 6.68; N, 4.88.

### Example 7: Application of the oxidative radical scission of hydroxyproline derivatives to the site-selective modification of tripeptides.

The tripeptide (66) (Scheme 20) was treated under the scission-oxidation conditions to afford the 4-oxohomoalanine derivative (42) in good yield. Remarkably, no epimerization products were detected.

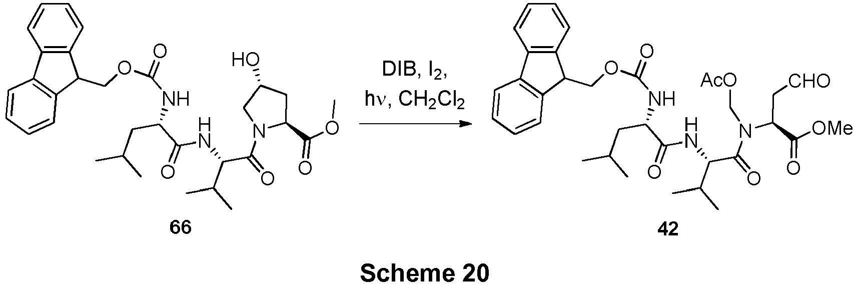

### Experimental procedure for example 7: Scission-Oxidation of Tripeptide (66).

*N-Fluorenylmethyloxycarbonyl-L-leucyl-L-valyl-4R-hydroxy-L-proline methyl ester* (66). A solution of Boc-Val-OH (700 mg, 3.23 mmol) and H-Hyp-OMe·HCl (1504 mg, 8.30 mmol) in dichloromethane (30 mL) at 0°C, was treated with DIPEA (3.5 mL, 20.1 mmol) and HBTU (2878 mg, 7.59 mmol) and stirred at 0°C for 1 h. The mixture was allowed to reach room temperature, and the stirring continued for 3 h, before pouring the solution into saturated aqueous NaHCO₃. Then the organic layer was washed with 10% aqueous HCl and brine, dried and concentrated as usual. The residue was purified by rotatory chromatography (hexane/EtOAc, 80:20), affording the intermediate dipeptide Boc-Val-Hyp-OMe (1051 mg, 3.05 mmol, 95%), as a white foam. The dipeptide was dissolved in CH₂Cl₂ (15 mL), cooled to 0°C, and treated with trifluoroacetic acid (15 mL). The mixture was allowed to reach room temperature, and the stirring continued for 2 h. Then the volatiles were removed under vacuum, and the residue was dissolved in CH₂Cl₂ (30 mL), cooled to 0°C, and treated with Cbz-Leu-OH (1140 mg, 4.30 mmol), DIPEA (2.95 mL, 16.94 mmol), HOBt·H₂O (722 mg, 4.71 mmol), and EDAC (904 mg, 4.71 mmol). After stirring for 1 h at 0°C, the mixture was allowed to reach room temperature, and the stirring continued for 20 h. After pouring the solution into saturated aqueous NaHCO₃, the organic layer was washed with 10% aqueous HCl and brine, dried and concentrated as usual. The residue was then purified by column chromatography on silica gel (hexane/EtOAc, 20:80), affording the tripeptide Fmoc-Leu-Val-Hyp-OMe (66) (1592 mg, 90%; global yield for the two steps 86%), as a white foam: [α]_{D} = -78.60 (*c* 0.16, CHCl₃); IR (CHCl₃) vₘₐₓ. 3428, 1744, 1718, 1651, 1646 cm⁻¹; ¹H NMR (500 MHz, CDCl₃, 26 °C) *δ*_{H} 0.87 (3H, d, *J* = 5.9 Hz, 2 x Me), 0.92 (3H, br d, *J* = 5.3 Hz, Me), 0.99 (3H, d, *J* = 5.3 Hz, Me), 1.54 (2H, m, 3"-H₂), 1.63 (1 H, m, 4"-H), 1.96 (1 H, m, 3-Hₐ), 2.09 (1 H, m, 3'-H), 2.33 (1 H, m, 3-H_{b}), 3.68 (1 H, m, 5-Hₐ), 3.71 (3H, s, OMe), 4.04 (1 H, m, 5-H_{b}), 4.16 (1 H, m, fluorenyl), 4.28 (2H, m, CHN + OCHₐ-fluorenyl), 4.38 (1H, brd, *J* = 7.4, 9.2 Hz, OCH_{b}fluorenyl), 4.40-4.50 (2H, m, 4-H + 2'-H), 4.64 (1 H, m, 2-H), 5.88 (1 H, br b, NH), 7.19 (1 H, br b, NH), 7.22-7.30 (2H, m, Ar), 7.36 (2H, dd, *J* = 7.4, 7.4 Hz, Ar), 7.55 (2H, br dd, *J* = 8.0, 8.8 Hz, Ar), 7.72 (2H, d, *J* = 7.5 Hz, Ar); ¹³C NMR (125.7 MHz, CDCl₃, 26 °C) *δ*_{C} 18.0 (CH₃), 19.1 (CH₃), 21.7 (CH₃), 23.0 (CH₃), 24.6 (CH), 30.8 (CH), 37.4 (CH₂), 41.1 (CH₂), 47.1 (CH), 52.2 (CH₃), 53.6 (CH), 55.9 (CH₂), 56.4 (CH), 57.8 (CH), 67.0 (CH₂), 70.1 (CH), 119.9 (2 x CH), 125.1 (2 x CH), 127.1 (2 x CH), 127.6 (2 x CH), 141.2 (2 x C), 143.6 (C), 143.8 (C), 156.3 (C), 170.9 (C), 172.4 (C), 173.3 (C); MS (EI) m/z (relative intensity) 579 (M⁺, <1), 435 (M⁺-Hyp-OMe, <1), 229 ([H-Val-Hyp-OMe - Me + H], 12), 211 (M⁺ - Fmoc - Hyp-OMe - H, 30), 178 ([fluorenyl-CH₂ - H]⁺, 100), 165 ([fluorenyl]⁺, 42); HRMS calcd for C₃₂H₄₁N₃O₇, 579.2945, found 579.2946; calculated for C₂₆H₃₁N₂O₄, 435.2284, found 435.2279; calculated for C₁₀H₁₆N₂O₄, 229.1188, found 229.1180; calculated for C₁₁H₁₉N₂O₂, 211.1447, found 211.1437; para C₁₄H₁₀, 178.0783, found 178.0776; calculated for C₁₃H₉, 165.0704, found 165.0699. Elemental analysis: Calculated for C₃₂H₄₁N₃O₇: C, 66.30; H, 7.13; N, 7.25; found C, 66.01; H, 7.00; N, 7.29.

*N-Acetoxymethyl-N-(N-fluorenylmethyloxycarbonyl-L-leucyl-L-valyl)-4-oxo-L-homoalanine methyl ester* (42). A solution of the tripeptide (66) (500 mg, 0.86 mmol) in dry DCM (10 mL), was treated with (diacetoxyiodo)benzene (554 mg, 1.72 mmol) and iodine (109 mg, 0.43 mmol). The reaction mixture was stirred at room temperature for 4 h, under irradiation with visible light. Usual work-up and concentration of the organic layer, followed by purification of the residue by column chromatography on silica gel (hexane/EtOAc, 60:40) afforded the aldehyde (42) (363 mg, 66%) as a yellowish gum. [α]_{D} = -53.48 (c 0.15, CHCl₃); IR (CHCl₃) v_{max.} 3424, 1744, 1724, 1665, 1509, 1503 cm⁻¹; ¹H NMR (500 MHz, CDCl₃, 26 °C) *δ*_{H} 0.86 (3H, d, *J* = 6.6 Hz, Me), 0.87-1.00 (9H, m, 3 x Me), 1.53 (1 H, m, 3"-Hₐ), 1.60-1.70 (2H, m, 3"-H_{b} + 4"-H), 1.98 (1 H, m, 3'-H), 2.08 (3H, s, Me), 2.98 (1 H, dd, *J* = 7.4, 18.6 Hz, CHₐ-CHO), 3.40 (1 H, dd, *J* = 5.2, 18.7 Hz, CH_{b}-CHO), 3.65 (3H, s, OMe), 4.15-4.25 (2H, m, 2"-H + fluorenyl), 4.30-4.50 (2H, m, OCH₂fluorenyl), 4.80-5.00 (2H, m, 2'-H + 2-H), 5.28 (1 H, d, *J* = 9.9 Hz, NH), 5.50 (1 H, d, *J* = 12.2 Hz, OCHₐN), 5.69 (1 H, d, *J* = 12.2 Hz, OCH_{b}N), 6.53 (1 H, d, *J* = 8.5 Hz, NH), 7.30 (2H, dd, *J* = 7.5, 7.8 Hz, Ar), 7.39 (2H, dd, *J* = 7.5, 7.5 Hz, Ar), 7.58 (2H, d, *J* = 7.5 Hz, Ar), 7.76 (2H, d, *J* = 7.5 Hz, Ar), 9.72 (1 H, s, CHO); ¹³C NMR (125.7 MHz, CDCl₃, 26 °C) *δ*_{C} 17.6 (CH₃), 19.3 (CH₃), 20.7 (CH₃), 22.1 (CH₃), 22.9 (CH₃), 24.6 (CH), 31.9 (CH), 41.5 (CH₂), 43.9 (CH₂), 47.2 (CH), 52.7 (CH₃), 53.5 (CH), 54.3 (CH), 55.6 (CH), 67.0 (CH₂), 72.4 (CH₂), 120.0 (2 x CH), 125.1 (2 x CH), 127.1 (2 x CH), 127.7 (2 x CH), 141.3 (2 x C), 143.7 (C), 143.9 (C), 156.1 (C), 169.7 (C), 170.5 (C), 172.0 (C), 173.5 (C), 198.4 (C); MS (EI) m/z (relative intensity) 435 (M⁺ - N(CH₂OAc)CH(CO₂Me)CH₂CHO, 1), 211 (H-Leu-Val + H, 8), 180 ([fluorenyl-CH₂ + H]⁺, 7), 178 ([fluorenyl-CH₂-H]⁺, 100); HRMS calcd for C₂₆H₃₁N₂O₄, 435.2284, found 435.2273; calculated for C₁₁H₁₉N₂O₂, 211.1447, found 211.1438; calculated for C₁₄H₁₂, 180.0939, found 180.0932; calculated for C₁₄H₁₀, 178.0783, found 178.0783. Elemental analysis: Calculated for C₃₄H₄₃N₃O₉: C, 64.03; H, 6.80; N, 6.59; found C, 63.90; H, 6.70; N, 6.58.

### Example 8: Application of the oxidative radical scission of hydroxyproline drivatives to the site-selective modification of tetrapeptides.

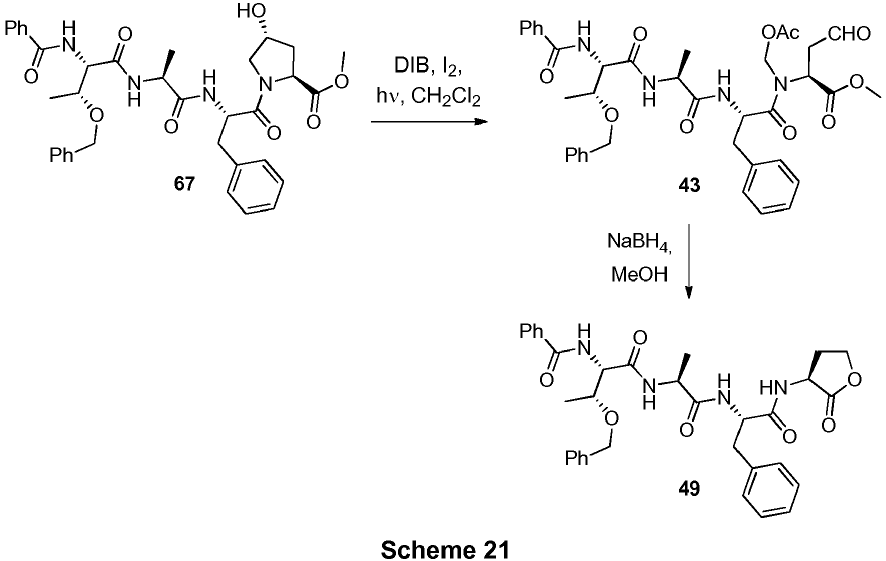

The tetrapeptide (67) (Scheme 21) was treated under the scission-oxidation conditions, affording the 4-oxohomoalanine derivative (43) in good yield; no epimerization products were detected. Then the aldehyde (43) underwent reduction and intramolecular cyclization to give homoserine lactone (49).

This example is remarkable because the tetrapeptide (67) presented two customizable units: the *N*-terminal threonine residue and the hydroxyproline unit. However, since the reactive hydroxyl group in threonine was protected, only the hydroxyproline unit reacted under the scission conditions. Both the scission and the reduction-lactonization steps were carried out under mild conditions, affording products with high optical purity.

### Experimental procedure for example 8: Scission-oxidation of tetrapeptide (67) followed by reduction-lactonization of derivative (43)

*N-Benzoyl-(O-benzyl-L-threonyl)-L-alanyl-L-phenylalanyl-L-trans-4-hydroxyproline methyl ester* (67): A solution of Boc-Thr(Bn)-Ala-Phe-OH (500 mg, 0.94 mmol) and H-Pro-OMe·HCl (206 mg, 1.13 mmol) in CH₂Cl₂ (15 mL) a 0 °C, was treated with DIPEA (0.3 mL, 1.72 mmol), HOBt·H₂O (159 mg, 1.04 mmol) and EDAC (199 mg, 1.04 mmol). The reaction mixture was stirred at 0°C for 1 h and then was allowed to reach room temperature, and the stirring continued for 20 h, before pouring the solution into saturated aqueous NaHCO₃. The organic layer was washed with 10% aqueous HCl and brine, dried and concentrated as usual. The residue was purified by column chromatography on silica gel (hexane/EtOAc, 10:90), affording the tetrapeptide Boc-Thr(Bn)-Ala-Phe-Hyp-OMe (507 mg, 82%), as a white foam. For easier product handling, the Boc protecting group was replaced by Bz. Thus, the tetrapeptide (507 mg, 0.78 mmol) was dissolved in CH₂Cl₂ (10 mL), cooled to 0 °C and treated with trifluoroacetic acid (10 mL) for 2h. Then the mixture was allowed to reach room temperature and the volatiles were removed under vacuum. The residue was treated with saturated aqueous NaHCO₃ (15 mL) and THF (10 mL) was added. The mixture was cooled in an ice bath, and BzCl (0.1 mL, 0.85 mmol) was injected dropwise. The solution was allowed to reach room temperature and stirred for 18 h, before pouring it into saturated aqueous NaHCO₃. After extraction with EtOAc, and usual drying and concentration of the organic layer, the residue was purified by recrystallization in EtOAc/hexane, affording the tetrapeptide Bz-Thr(Bn)-Ala-Phe-Hyp-OMe (67) (389 mg, 77%; global yield for the two steps 63%) as a white solid: m.p. 200-203 °C; [α]_{D} = -40.9 (c 0.19, CHCl₃); ¹H NMR (500 MHz, CDCl₃, 26 °C) *δ*_{H} 1.20 (3H, d, *J* = 6.3 Hz, Me), 1.22 (3H, d, *J* = 6.9 Hz, Me), 1.88 (1 H, ddd, *J* = 4.1, 8.8, 13.3 Hz, 3-Hₐ), 2.26 (1 H, dd, *J* = 7.9, 13.3 Hz, 3-H_{b}), 2.68 (1 H, br b, OH), 2.89 (1 H, dd, *J* = 6.0, 13.9 Hz, 3'-Hₐ), 3.10 (1 H, dd, *J* = 7.3, 13.6 Hz, 3'-H_{b}), 3.16 (1 H, dd, *J* = 3.5, 11.0 Hz, 5-Hₐ), 3.70 (3H, s, OMe), 3.74 (1H, d, *J* = 11.3 Hz, 5-H_{b}), 4.20 (1H, m, 3"'-H/CH(Me)OR), 4.36 (1 H, m, 4-H), 4.43 (1 H, m, 2"-H), 4.56 (1 H, d, *J* = 8.5 Hz, 2-H), 4.60 (1 H, d, *J* = 11.7 Hz, OCHₐN), 4.71 (1 H, d, *J* = 11.4 Hz, OCH_{b}N), 4.82 (1 H, dd, *J* = 3.8, 6.6 Hz, 2"'-H), 4.89 (1 H, ddd, *J* = 6.7, 7.3, 7.6 Hz, 2'-H), 7.06 (1 H, br d, *J* = 8.0 Hz, NH), 7.15-7.40 (12H, m, 2NH + 10Ar), 7.44 (2H, dd, *J =* 7.4, 7.8 Hz, Ar), 7.52 (1 H, dd, *J* = 7.3, 7.5 Hz, Ar), 7.81 (2H, d, *J* = 7.3 Hz, Ar); ¹³C NMR (125.7 MHz, CDCl₃, 26 °C) *δ*_{C} 15.5 (CH₃), 18.1 (CH₃), 37.4 (CH₂), 38.2 (CH₂), 49.4 (CH), 52.2 (CH + CH₃), 55.2 (CH₂), 56.7 (CH), 58.0 (CH), 70.1 (CH), 71.8 (CH₂), 74.7 (CH), 126.9 (CH), 127.3 (2 x CH), 127.9 (2 x CH), 128.0 (CH), 128.4 (2 x CH), 128.6 (2 x CH), 128.7 (2 x CH), 129.8 (2 x CH), 132.0 (CH), 133.6 (C), 136.0 (C), 137.9 (C), 167.6 (C), 169.6 (C), 169.9 (C), 171.6 (C), 172.4 (C); MS (EI) m/z (relative intensity) 658 (M⁺ + H, <1), 105 (Bz, 100), 91 (CH₂Ph, 60), 77 (Ph, 66); HRMS calcd for C₃₆H₄₂N₄O₈, 658.3003, found 658.2990; calculated for C₇H₅O, 105.0340, found 105.0341; calculated for C₆H₅, 77.0391, found 77.0392. Elemental analysis: Calculated for C₃₆H₄₁N₄O₈: C, 65.64; H, 6.43; N, 8.51; found C, 65.38; H, 6.54; N, 8.73.

*N-Acetoxym eth yl-N-(N-benzo yl-(O-benzyl-L-threon yl)-L-alan yl-L-phenylalanyl)-4-oxo-L-homoalanine methyl ester* (43): A solution of the tetrapeptide (67) (330 mg, 0.5 mmol) in dry CH₂Cl₂ (10 mL) was treated with DIB (322 mg, 1.0 mmol) and iodine (127 mg, 0.5 mmol). The mixture was stirred at 26 °C for 4 h, under irradiation with visible light. After usual work-up and concentration of the organic layer, the residue was purified by column chromatography on silica gel (hexane/EtOAc, 40:60) affording the aldehyde (43) (186 mg, 52%) as a white foam: [α]_{D} = -31.64 (c 0.18, CHCl₃); ¹H NMR (500 MHz, CDCl₃, 26 °C) *δ*_{H} 1.20 (3H, d, *J* = 6.3 Hz, Me), 1.30 (3H, d, *J* = 7.0 Hz, Me), 1.98 (3H, s, Ac), 2.76 (1 H, dd, *J* = 7.4, 18.6 Hz, 3-Hₐ), 2.93 (1 H, dd, *J* = 5.9 Hz, 13.4 Hz, 3'-Hₐ), 2.99 (1 H, dd, *J* = 8.2, 13.3 Hz, 3'-H_{b}), 3.33 (1 H, dd, *J* = 5.4, 18.9 Hz, 3-H_{b}), 3.63 (3H, s, OMe), 4.27 (1 H, m, 3"'-H/CHOR), 4.47 (1 H, dddd, *J* =7.0, 7.2, 7.3, 7.3 Hz, 2"-H), 4.66 (1 H, d, *J* = 11.7 Hz, OCHₐPh), 4.69 (1 H, dd, *J* = 5.4, 7.3 Hz, 2-H/CHNR₂), 4.76 (1 H, d, *J* = 11.7, OCH_{b}Ph), 4.79 (1 H, dd, *J* = 3.5, 6.3 Hz, 2"'-H/CHN), 5.15 (1 H, d, *J =* 12.3 Hz, OCHₐN), 5.26 (1 H, m, 2'-H), 5.40 (1 H, d, *J* = 12.3 Hz, OCH_{b}N), 6.85 (1 H, d, *J* = 7.9 Hz, NH), 7.07 (1 H, d, *J* = 7.6 Hz, NH), 7.13 (2H, d, *J =* 7.2 Hz, Ar), 7.18-7.26 (4H, m, 3Ar + NH), 7.29 (1 H, dd, *J =* 7.3, 7.3 Hz, Ar), 7.35 (2H, dd, *J* = 7.3, 8.2 Hz, Ar), 7.40 (2H, d, *J* = 7.7 Hz, Ar), 7.45 (2H, dd, *J* = 7.3, 7.9 Hz, Ar), 7.53 (1 H, dd, *J* = 7.3, 7.6 Hz, Ar), 7.83 (2H, d, *J =* 7.9 Hz, Ar), 9.61 (1 H, s, CHO); ¹³C NMR (125.7 MHz, CDCl₃, 26 °C) *δ*_{C} 15.3 (CH₃), 18.0 (CH₃), 20.6 (CH₃), 39.3 (CH₂), 43.8 (CH₂), 49.0 (CH), 50.6 (CH), 52.6 (CH₃), 55.6 (CH), 56.4 (CH), 71.7 (CH₂), 72.0 (CH₂), 74.3 (CH), 127.1 (3 x CH), 127.9 (3 x CH), 128.5 (5 x CH), 129.4 (3 x CH), 131.9 (CH), 133.6 (C), 135.5 (C), 137.9 (C), 167.3 (C), 169.2 (C), 169.5 (C), 170.4 (C), 171.0 (C), 172.8 (C), 198.3 (CH); EM (ESI) m/z (relative intensity) 739 ([M + Na]⁺, 60), HRMS calcd for C₃₈H₄₄N₄O₁₀Na 739.2955, found 739.2955. Elemental analysis: Calculated for C₃₈H₄₄N₄O₁₀: C, 63.67; H, 6.19; N, 7.82; found C, 63.87; H, 6.32; N, 8.13.

*N-(N-Benzoyl-(O-benzyl-L-threonyl)-L-alanyl-L-phenylalanyl)-L-homoserine lactone* (49): A solution of the aldehyde (43) (36 mg, 0.05 mmol) in dry methanol (1.5 mL) was treated with NaBH₄ (3 mg, 0.08 mmol) and the mixture was stirred at 26 °C for 20 h. Then it was poured into water and extracted with EtOAc. The organic layer was dried and concentrated as usual, and the residue was purified by rotatory chromatography (hexane/EtOAc, 40:60) yielding the product (49) (22 mg, 72%) as a yellowish foam: [α]_{D} =-19.44 (*c* 0.42, CHCl₃); ¹H NMR (500 MHz, CDCl₃, 26 °C) *δ*_{H} 1.32 (3H, d, *J* = 6.3 Hz, Me), 1.33 (3H, d, *J* = 7.3 Hz, Me), 2.26 (1 H, m, 4-Hₐ), 2.48 (1 H, m, 4-H_{b}), 3.03 (1 H, dd, *J* = 8.5, 13.9 Hz, 3'-Hₐ), 3.23 (1 H, dd, *J* = 5.7, 13.9 Hz, 3'-H_{b}), 4.20 (1 H, m, 3"'-H/CHOBn), 4.32 (1 H, m, 5-Hₐ), 4.40 (1 H, m, 2"'-H/CHN), 4.45 (1 H, m, 4-H_{b}), 4.58 (1 H, d, *J* = 11.4 Hz, OCHₐPh), 4.59-4.63 (2H, m, 2'-H + 3-H), 4.69 (1 H, d, *J* = 11.4 Hz, OCH_{b}Ph), 4.75 (1 H, d, *J* = 4.1 Hz, 2"'-H/CHN), 7.19 (1 H, m, Ar), 7.22-7.25 (4H, m, Ar), 7.32 (1 H, dd, *J =* 7.0, 7.2 Hz, Ar), 7.36 (2H, dd, *J* = 7.6, 7.8 Hz, Ar), 7.41 (2H, d, *J* = 6.9 Hz, Ar), 7.53 (2H, dd, J = 7.6, 7.9 Hz, Ar), 7.62 (1 H, dd, *J* = 7.2, 7.6 Hz, Ar), 7.91 (2H, d, *J* = 7.3 Hz); ¹³C NMR (125.7 MHz, CDCl₃, 26 °C) *δ*_{C} 15.6 (CH₃), 16.5 (CH₃), 28.1 (CH₂), 37.1 (CH₂), 48.5 (CH), 49.4 (CH), 54.5 (CH), 58.3 (CH), 65.8 (CH₂), 71.2 (CH₂), 75.0 (CH), 126.4 (CH), 127.1 (2 x CH), 127.5 (CH), 127.8 (2 x CH), 128.0 (4 x CH), 128.2 (2 x CH), 129.0 (2 x CH), 131.7 (CH), 133.6 (C), 136.8 (C), 138.2 (C), 169.0 (C), 171.0 (C), 171.8 (C), 173.0 (C), 175.6 (C); MS (EI) m/z (relative intensity) 614 (M⁺, <1), 296 (BzNHCH(CO)CH(Me)OBn, 2), 105 (([PhCO]⁺, 100), 91 ([PhCH₂]⁺, 81); HRMS calcd for C₃₄H₃₈N₄O₇, 614.2741, found 614.2742; calculated for C₁₈H₁₈NO₃, 296.1287, found 296.1286; calculated for C₇H₅O, 105.0340, found 105.0345; calculated for C₇H₇, 91.0548, found 91.0548. Elemental analysis: Calculated for C₃₄H₄₁NO₉: C, 66.43; H, 6.23; N, 9.11; found C, 66.09; H, 6.62; N, 9.10.

## Claims

1. Procedure for the selective modification of a hydroxylate cyclic amino acid derivative with the formula (36), to generate a compound with the formula (37), and conversion of the latter into amino acid derivatives with the formula (38) 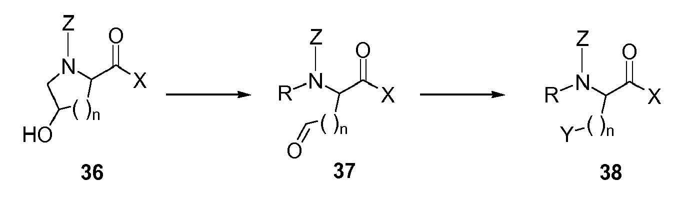 where
Z represents the *N*-substituent of the amino acid derivative, and is selected from hydrogen, alkyl, acyl, carbamoyl, amino acyl, and peptidyl;
R represents one of the *N*-substituents of products (37) or (38) and is selected from H and alkyl;
n is an integer between 1 and 5;
X represents the carboxyl group substituent of the hydroxyproline (36) or the modified amino acid derivatives (37) or (38), and is selected from H, hydroxy, alkoxy, amino, amido, alkyl, acyl, aryl, amino acyl, and peptidyl;
Y represents groups of the lateral chain of the modified amino acid in compound (38), and is selected from carbonyl, carboxyl, CH₂OH, CH₂-amino, alkyl, acyl, CH₂-amino acyl, and CH₂-peptidyl, or alternatively is alkyl or acyl and is directly attached to a group X selected from O, NH or N-alkyl,
and is **characterized in that**:
a) the conversion of substrate with formula (36) into the product with formula (37) uses a radical scission step where the configuration of the α-stereogenic center of the modified amino acid is retained, and therefore, the resulting products (37) are obtained with high optical purity,
b) the conversion of the compound with the formula (37) into the product with the formula (38) is carried out using a reaction selected from, but not limited to, reductive amination, reduction-lactonization, or addition to the carbonyl group, including the Horner-Wadsworth-Emmons reaction.

2. Procedure for the selective modification of an amino acid derivative with formula (36) into a derivative (37) according to claim 1, **characterized in that** the radical scission is oxidative

3. Procedure for the selective modification of a compound with formula (36) according to any one of claims 1 or 2, **characterized in that** n = 1 and thus the customizable amino acid is a 4-hydroxyproline derivative

4. Procedure for the selective modification of a compound of general formula (36) according to any one of claims 1 to 3, wherein Z is acyl or carbamoyl and X is alkoxy or amino, and the product (37) consists of a single amino acid residue

5. Procedure for the selective modification of a compound with formula (36) according to any one of claims 1 to 3, wherein either Z or X, or both Z and X are amino acid or peptidyl, and the product (37) is a peptide

6. Procedure for the selective modification of a compound with formula (36) according to any one of claims 1 to 5, wherein a cationic intermediate is generated and trapped by *O*-nucleophiles, to give a compound (37) **characterized in that** N-R is N-H or a N,O-acetal, particularly NCH₂OAc.

7. Procedure for the selective modification of a compound with formula (36) according to any one of the preceding claims, **characterized in that** the reagent for the oxidative radical scission step is (diacetoxyiodo)benzene-iodine.

8. Procedure for the selective modification of a compound with formula (36) according to any one of the preceding claims, **characterized in that** the radical scission step is activated with an energy supply selected from, but not limited to, heating, irradiation with light or irradiation with microwaves.

9. Procedure for the selective modification of a compound with formula (36) according to claim 8, **characterized in that** it uses visible light irradiation as activation energy.

10. Procedure for the selective modification of a compound with formula (36) according to any one of claims 1 to 9, **characterized in that** the solvent used in the radical scission step is aprotic.

11. Procedure for the selective modification of an amino acid derivative according to claim 1, **characterized in that** the group N-R in compound (37) is an N,O-acetal, preferably NCH₂OAc, and undergoes hydrolysis to give a compound (38) wherein N-R is N-H.

12. Procedure for the selective modification of an amino acid derivative according to claim 1, **characterized in that** the group N-R in compound (37) is an N,O-acetal, preferably NCH₂OAc, and undergoes reduction to give a compound (38) wherein N-R is N-Me.

13. Procedure for the selective modification of an amino acid derivative according to claim 1, **characterized in that** the group N-R in compound (37) is an N,O-acetal, preferably NCH₂OAc, and undergoes addition of nucleophiles to the acetal group.

14. Compound of general formula (37), obtained by a method as defined in any one of claims 1-10.

15. Compound of general formula (37) according to claim 14, **characterized in that** it is selected from the compounds (39) to (43).

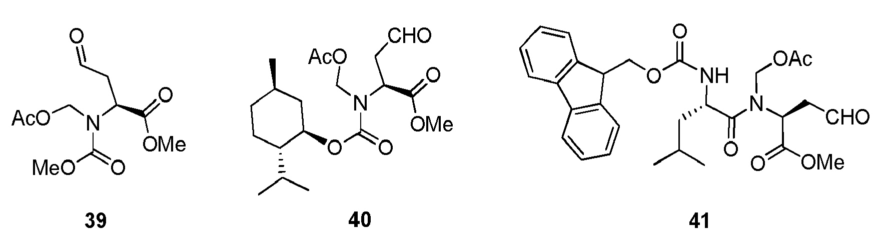

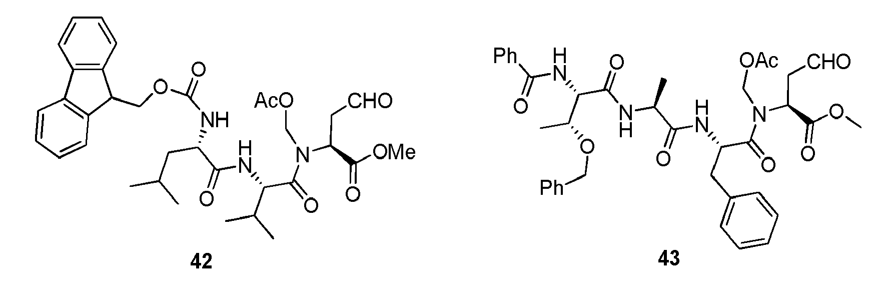

16. Compound of general formula (38), obtained by a method as defined in any one of claims 1 and 11 to 13, from a compound (37) as described in claim 14.

17. Compound of general formula (38) according to claim 16, wherein Y is alkyl and is directly attached to a group X selected from O, NH or N-alkyl, and such lactone or lactam rings are formed by reduction or reductive amination of compounds with the formula (37).

18. Compound of general formula (38) according to claim 17, **characterized in that** it is selected from the compounds (44) to (49).

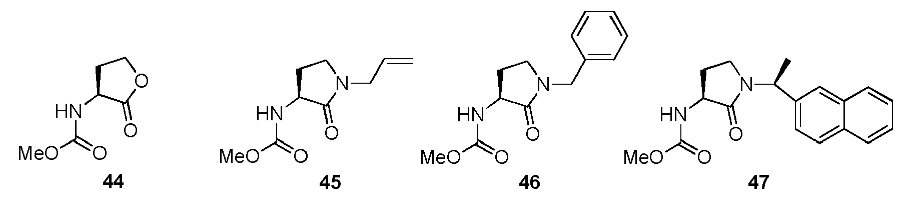

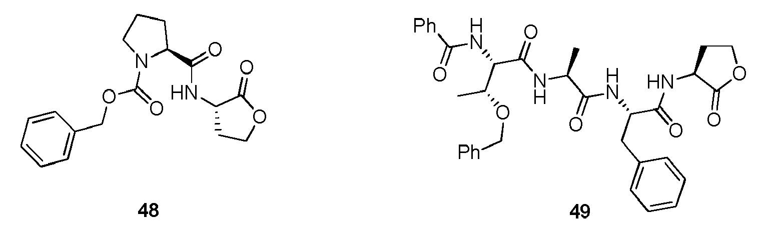

19. Compound with formula (38) according to claim 16, wherein Y = methyleneamino, generated by reductive amination of the compounds with formula (37).

20. Compound with general formula (38) according to claim 19, **characterized in that** it is selected from the compounds with formula (50) to (57).

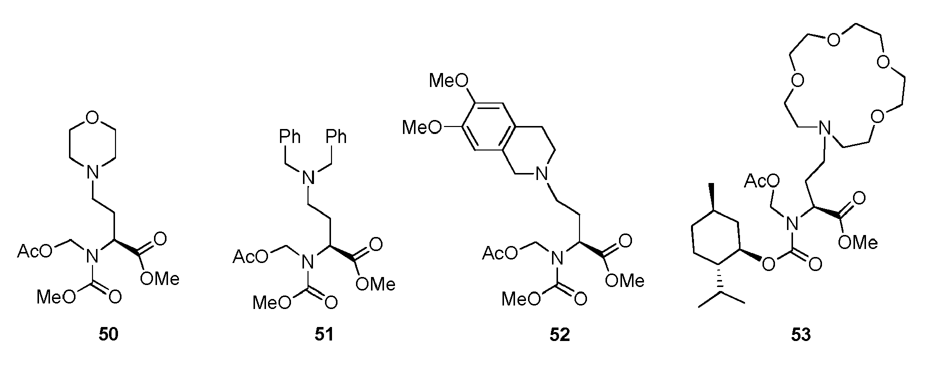

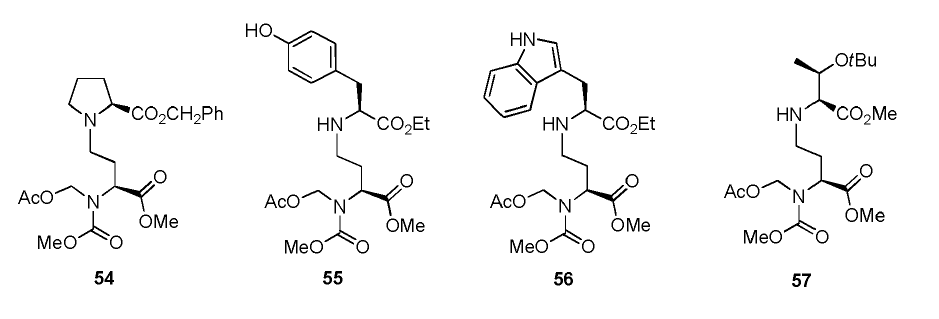

21. Compound with general formula (38) according to claim 16, wherein Y = alkenyl or alkyl, and is obtained from a compound with formula (37) by a Horner-Wadsworth-Emmons reaction followed in some cases by reduction of the resulting olefin.

22. Compound with general formula (38) according to claim 21, **characterized in that** it is selected from the compounds with formula (58) to (60).

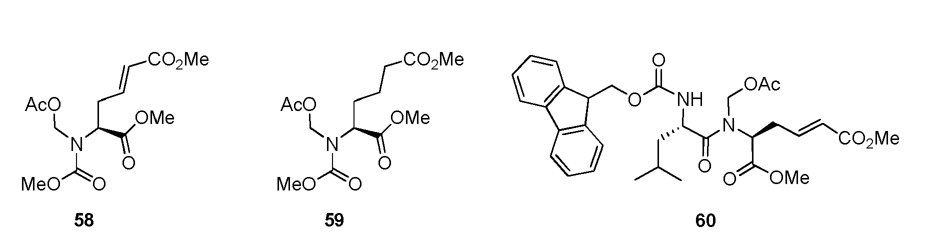

23. Compound with general formula (38) according to claim 16, wherein R = Me, and is obtained from a compound with formula (37) wherein N-R is an N,O-acetal, preferably NCH₂OAc.

24. Compound with the general formula (38) according to claim 23, **characterized in that** it is selected from the compounds with formula (61) or (62).

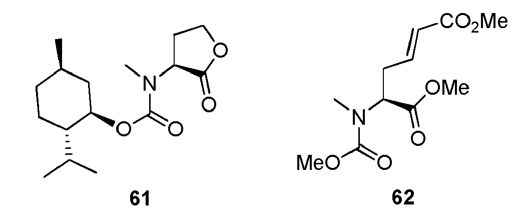

25. Compound with the formula (37) according to claims 14, for use thereof in the discovery of new catalytic or bioactive compounds and drugs.

26. Compound with the general formula (38) according to any one of claims 16 to 24, for use thereof in the discovery of new catalytic or bioactive compounds and drugs, or medical probes for molecular imaging.

27. Compound with the formula (38) according to any one of claims 16 to 22, wherein N-R is an N,O-acetal, preferably NCH₂OAc, for use thereof in the preparation of N-methyl or N-alkyl amino acid derivatives, by reduction or addition of nucleophiles to the N,O-acetal group.

28. Compound with the general formula (37) or (38), according to any one of claims 14 to 24, for use thereof in the preparation of a larger compound, such as a peptide, a depsipeptide or a conjugate with other compounds.
